# EUROPEAN PATENT SPECIFICATION

(11) **EP 1 555 984 B1**
(45) Date of publication and mention of the grant of the patent: **12.03.2008**
(21) Application number: 03773718.6
(22) Date of filing: 16.10.2003
(51) Int. Cl.: B01F 17/00

(54) **PROCESS FOR DISSOLVING LIPOPHILIC COMPOUNDS, AND COSMETIC COMPOSITION.**
VERFAHREN ZUM AUFLÖSEN LIPOPHILER VERBINDUNGEN, UND KOSMETISCHE ZUSAMMENSETZUNG.
PROCEDE DE DISSOLUTION DE COMPOSES LIPOPHILES DANS UNE SOLUTION AQUEUSE AVEC DES COPOLYMERES SEQUENCES AMPHIPHILES, ET COMPOSITIONS COSMETIQUES

(30) Priority: 21.10.2002 FR 0213101; 12.12.2002 US 432619 P
(43) Date of publication of application: 27.07.2005
(73) Proprietor: L'ORÉAL, 75008 Paris (FR)
(72) Inventor: SIMONNET, Jean-Thierry, F-94240 Cachan (FR)
(74) Representative: Dodin, Catherine
(86) International application number: PCT/EP2003/013050
(87) International publication number: WO 2004/035013

(56) References cited:
- EP-A- 0 870 781
- EP-A- 1 125 574
- EP-A- 1 127 570
- EP-A- 1 281 395
- EP-A- 1 302 197
- WO-A-00/12660
- WO-A-01/12718
- WO-A-99/47589
- FR-A- 2 085 902
- FR-A- 2 140 977
- FR-A- 2 155 901
- US-B1- 6 322 805

## Description

The present invention relates to a process for dissolving lipophilic compounds with amphiphilic block copolymers. The invention also relates to novel cosmetic compositions resulting from the implementation of this process.

A large number of lipophilic molecules have very low solubilities in cosmetic solvents and have a tendency to recrystallize rapidly. Moreover, it is sought to avoid the presence of oily solvents for sensory reasons, or for reasons of compatibility in the case of dissolving two molecules with respective solvents that are incompatible with each other, or else for reasons of harmlessness if it is not desired to use solvents that can modify the barrier function of the skin.
Thus, in the case of sunscreens, in order to have substantial photoprotection, large proportions of solvent oils are necessary. This may be harmful to the final cosmetic feel of the composition containing them: oily, greasy feel. Formulation solutions that can increase the solubility of these sunscreens in water while at the same time avoiding the use of solvents such as ethanol and isopropanol, which are harmful to the integrity of the barrier function of the skin, have thus been sought.
It is known practice to combine certain compounds with lipophilic molecules, especially DHEA, in order to improve their solubility. However, none of these compounds is a block copolymer.
It is known practice to encapsulate lipophilic compounds in micelles of block copolymers, for example poly(ethylene oxide-propylene oxide) diblock or triblock copolymers. However, these block copolymers do not satisfactorily dissolve the lipophilic compound.
It is thus seen that it is necessary to develop families of readily available copolymers, enabling a satisfactory dissolution of the lipophilic compound by encapsulation in micelles that are stable at room temperature.

The Applicant has just discovered, surprisingly, that by combining a lipophilic compound with an effective amount of at least one block amphiphilic copolymer comprising at least one ionic or nonionic hydrophilic polymer block and at least one particular hydrophobic polymer block, the lipophilic compound can be satisfactorily dissolved, thus solving the drawbacks of the prior art.

One subject of the present invention is thus a process for dissolving lipophilic compounds in an aqueous phase using block copolymers.
A subject of the invention is also a cosmetic composition comprising a lipophilic compound and a block copolymer in aqueous phase.
Other characteristics, aspects and advantages of the invention will emerge on reading the detailed description that follows.

The process for dissolving at least one lipophilic compound in aqueous phase in accordance with the invention is characterized in that it comprises the step of associating (physical combination) said lipophilic compound with an effective amount of at least one amphiphilic block copolymer comprising at least one ionic and/or at least one nonionic hydrophilic polymer block, and at least one hydrophobic polymer block obtained from at least one hydrophobic monomer chosen from :
- styrene and its derivatives such as 4-butylstyrene,
- vinyl acetate of formula CH₂=CH-OCOCH₃,
- vinyl ethers of formula CH₂=CHOR in which R is a linear or branched, saturated or unsaturated hydrocarbon-based radical containing from 1 to 6 carbon atoms,
- acrylonitrile,
- vinyl chloride and vinylidene chloride,
- caprolactam,
- alkenes such as ethylene, propylene, butylene and butadiene,
- alkylene oxides containing at least 4 carbon atoms and preferably from 4 to 6 carbon atoms,
- silicone derivatives producing, after polymerization, silicone polymers such as polydimethylsiloxane,
- hydrophobic vinyl monomers of formula (A) below: 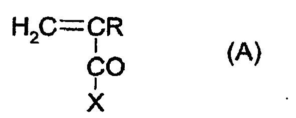
in which:
- R is chosen from H, -CH₃, -C₂H₅ and -C₃H₇,
- X is chosen from:
   - alkyl oxides of -OR' type in which R' is a linear, cyclic or branched, saturated or unsaturated hydrocarbon-based radical containing from 1 to 22 carbon atoms, optionally substituted with a halogen atom (iodine, bromine, chlorine or fluorine); a sulphonic group (-SO₃⁻), a sulphate group (-SO₄⁻), a phosphate group (-PO₄H₂⁻); a hydroxyl group (-OH); a primary amine group (-NH₂); a secondary amine group (-NHR₁), a tertiary amine group (-NR₁R₂) or a quaternary amine group (-N⁺R₁R₂R₃) with R₁, R₂ and R₃ being, independently of each other, a linear or branched, saturated or unsaturated hydrocarbon-based radical containing from 1 to 22 carbon atoms, with the proviso that the sum of the carbon atoms of R' + R₁ + R₂ + R₃ does not exceed 22; R' may also be a perfluoroalkyl radical, preferably containing from 1 to 18 carbon atoms;
   - -NH₂, -NHR' and -NR'R" groups in which R' and R" are, independently of each other, linear, cyclic or branched, saturated or unsaturated hydrocarbon-based radicals containing from 1 to 22 carbon atoms, with the proviso that the total number of carbon atoms of R' + R" does not exceed 22, R' and R" optionally being substituted with a halogen atom (iodine, bromine, chlorine or fluorine); a hydroxyl group (-OH); a sulphonic group (-SO₃⁻); a sulphate group (-SO₄⁻); a phosphate group (-PO₄H₂⁻); a primary amine group (-NH₂); a secondary amine group (-NHR₁), a tertiary amine group (-NR₁R₂) and/or a quaternary amine group (-N⁺R₁R₂R₃) with R₁, R₂ and R₃ being, independently of each other, a linear or branched, saturated or unsaturated hydrocarbon-based radical containing from 1 to 22 carbon atoms, with the proviso that the sum of the carbon atoms of R' + R" + R₁ + R₂ + R₃ does not exceed 22; R' and R" may also be perfluoroalkyl radicals, preferably containing from 1 to 18 carbon atoms.

The expression "effective amount of amphiphilic copolymer" means an amount that is sufficient for the copolymer to dissolve by itself the lipophilic compound in the aqueous phase, without recrystallization or creaming (for molecules that are oily at room temperature) of the said lipophilic compound.

The block copolymers used in the process according to the invention are especially those that can form micelles or lyotropic liquid crystal phases of lamellar, cubic (direct or inverse) or hexagonal (direct or inverse) type on contact with water. They are especially of diblock (A-B), triblock (B-A-B) or triblock (A-B-A) type, A corresponding to the ionic and/or nonionic hydrophilic polymer block and B to the hydrophobic polymer block.

The ionic hydrophilic polymer block may be obtained from water-soluble monomers or salts thereof, which may be chosen from:
- (meth)acrylic acid,
- acrylamido-2-methylpropanesulphonic (AMPS) acid,
- styrenesulphonic acid,
- vinylsulphonic acid and (meth)allylsulphonic acid,
- vinylphosphonic acid,
- maleic anhydride,
- itaconic acid,
- dimethyldiallylammonium chloride,
- quatemized dimethylaminoethyl methacrylate (DMAEMA),
- (meth)acrylamidopropyltrimethylammonium chloride (APTAC and MAPTAC),
- methylvinylimidazolium chloride,
- hydrophilic vinyl monomers of formula (A) below: 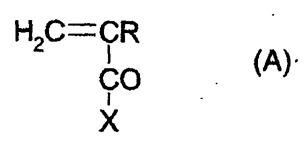 in which:
- R is chosen from H, -CH₃, -C₂H₅ and -C₃H₇,
- X is chosen from:
   - alkyl oxides of -OR' type in which R' is a linear, cyclic or branched, saturated or unsaturated hydrocarbon-based radical containing from 1 to 6 carbon atoms, substituted with at least one sulphonic group (-SO₃⁻) and/or sulphate group (-SO₄⁻) and/or phosphate group (-PO₄H₂⁻) and/or quaternary amine group (-N⁺R₁R₂R₃) with R₁, R₂ and R₃ being, independently of each other, a linear or branched, saturated or unsaturated hydrocarbon-based radical containing from 1 to 6 carbon atoms, with the proviso that the sum of the atoms of R' + R₁ + R₂ + R₃ does not exceed 6; the radical R' being optionally substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁, R₂ being, independently of each other, a linear or branched, saturated or unsaturated hydrocarbon-based radical containing from 1 to 6 carbon atoms, with the proviso that the sum of the carbon atoms of R' + R₁ + R₂ does not exceed 6;
   - -NH₂, -NHR' and -NR'R" groups in which R' and R" are, independently of each other, linear, cyclic or branched, saturated or unsaturated hydrocarbon-based radicals containing from 1 to 6 carbon atoms, with the proviso that the total number of carbon atoms of R' + R" does not exceed 6, R' and/or R" being substituted with at least one sulphonic group (-SO₃⁻) and/or sulphate group (-SO₄⁻) and/or phosphate group (-PO₄H₂⁻) and/or quaternary amine group (-N⁺R₁R₂R₃) with R₁, R₂ and R₃ being, independently of each other, a linear or branched, saturated or unsaturated hydrocarbon-based radical containing from 1 to 6 carbon atoms, with the proviso that the sum of the atoms of R' + R₁ + R₂ + R₃ does not exceed 6; the radicals R' and/or R" being optionally substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁, R₂ being, independently of each other, a linear or branched, saturated or unsaturated hydrocarbon-based radical containing from 1 to 6 carbon atoms, with the proviso that the sum of the carbon atoms of R' + R" + R₁ + R₂ does not exceed 6.

The ionic hydrophilic polymer block may also be polyethyleneimine.

The ionic hydrophilic polymer block may also be obtained from hydrophobic monomers, the said hydrophobic monomers being present in an amount that is small enough for the ionic hydrophilic polymer block to be soluble in water. The hydrophobic monomers may be chosen from:
- styrene and its derivatives such as 4-butylstyrene, .
- vinyl acetate of formula CH₂=CH-OCOCH₃,
- vinyl ethers of formula CH₂=CHOR in which R is a linear or branched, saturated or unsaturated hydrocarbon-based radical containing from 1 to 6 carbon atoms,
- acrylonitrile,
- vinyl chloride and vinylidene chloride,
- hydrophobic vinyl monomers of formula (A) below: 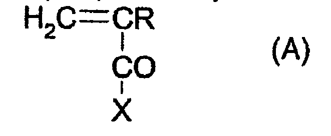
in which:
- R is chosen from H, -CH₃, -C₂H₅ or -C₃H₇,
- X is chosen from:
   - alkyl oxides of -OR' type in which R' is a linear or branched, saturated or unsaturated hydrocarbon-based radical containing from 1 to 6 carbon atoms,
   - -NH₂, -NHR' and -NR'R" groups in which R' and R" are, independently of each other, linear or branched, saturated or unsaturated hydrocarbon-based radicals containing from 1 to 6 carbon atoms, with the proviso that the total number of carbon atoms of R' + R" does not exceed 6.

The ionic hydrophilic polymer block is preferably chosen from (meth)acrylic acid.

Said ionic hydrophilic polymer block could be neutralized, partly or totally, by using an organic or non organic base, such as sodium, ammonium, lithium, calcium, magnesium, salts substituted by 1-4 alkyl groups containing 1-15 carbon atoms. Other suitable bases are mono-, di- or triéthanolamine, aminoethylpropanediol, N-methyl glucamine, or basic aminoacids such as arginine or lysine.

The nonionic hydrophilic polymer block may be of polyoxyalkylenated type, for instance polyoxyethylene. It may also be polyvinylpyrrolidone (PVP). The nonionic hydrophilic polymer block may also be obtained from water-monomers chosen from:
- (meth)acrylamide,
- N-vinylacetamide and N-methyl-N-vinylacetamide,
- N-vinylformamide and N-methyl-N-vinylformamide,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula CH₂=CHOH,
- glycidyl (meth)acrylate,
- hydrophilic vinyl monomers of formula (A) below: 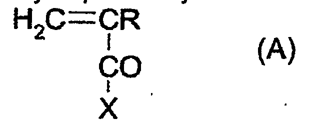
in which:
- R is chosen from H, -CH₃, -C₂H₅ and -C₃H₇,
- X is chosen from:
   - alkyl oxides of -OR' type in which R' is a linear or branched, saturated or unsaturated hydrocarbon-based radical containing from 1 to 6 carbon atoms, optionally substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁ and R₂ being, independently of each other, a linear or branched, saturated or unsaturated hydrocarbon-based radical containing from 1 to 6 carbon atoms, with the proviso that the sum of the carbon atoms of R' + R₁ + R₂ does not exceed 6;
   - -NH₂, -NHR' and -NR'R" groups in which R' and R" are, independently of each other, linear or branched, saturated or unsaturated hydrocarbon-based radicals containing from 1 to 6 carbon atoms, with the proviso that the total number of carbon atoms of R' + R" does not exceed 6, R' and R" optionally being substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁ and R₂ being, independently of each other, a linear or branched, saturated or unsaturated hydrocarbon-based radical containing from 1 to 6 carbon atoms, with the proviso that the sum of the carbon atoms of R' + R" + R₁ + R₂ does not exceed 6.

The nonionic hydrophilic polymer block is preferably chosen from polyethylene oxide or polyoxyethylene (PEO) and polyvinylpyrrolidone (PVP).

Preferably, the hydrophilic polymer block is nonionic.

Preferably, the hydrophilic polymer block is an homo-polymer.

The hydrophobic polymer block is obtained from at least one of the monomers cited above. It may also be obtained from hydrophilic monomers, being present in an amount that is small enough for the hydrophobic polymer block to be hydrophobic.

The hydrophilic monomers may be chosen from the above-mentioned water-soluble monomers suitable from obtaining nonionic hydrophilic polymer block or ionic hydrophilic polymer block.

Preferably, the hydrophobic polymer block is an homo-polymer.

The hydrophobic polymer block is preferably obtained from at least one hydrophobic monomers chosen from styrene, tert-butylstyrene, methyl methacrylate, ethyl (meth)acrylate, n-butyl (meth)acrylate, tert-butyl (meth)acrylate, cyclohexyl acrylate, isobornyl acrylate, 2-ethylhexyl acrylate, ethyl perfluorooctyl acrylate, trifluoromethyl (meth)acrylate, polybutylene oxide or polyoxybutylene (POB), butadiene, ethylene, propylene and butylene.

The block copolymer is preferably chosen from the following block copolymers:
- polystyrene/polyoxyethylene
- polymethyl methacrylate/polyoxyethylene
- polybutyl methacrylate/polyoxyethylene
- polyoxybutylene/polyoxyethylene
- polyethylene/polyoxyethylene
- polyoxyethylene/polyoxybutylene/polyoxyethylene.

The molecular weight of the block copolymer may be between 1 000 and 500 000, preferably from 2000 to 100 000.

The molecular weight of the hydrophilic block may be between 600 and 300 000, preferably from 1200 to 60 000.

The molecular weight of the hydrophobic block may be between 400 and 200 000, preferably from 800 to 40 000.

The weight ratio A/B between the hydrophilic block A and the hydrophobic block B ratio may be between 1/100 and 50/1.

In addition, the weight concentration ratio between the lipophilic compound and the amphiphilic block copolymer used according to the invention is generally between 0.005 and 0.5 and preferably between 0.005 and 0.2.

The lipophilic compounds are preferably those with a solubility in water of less than 0.2% by weight. The measurement is performed by UV spectrometry or HPLC on an aqueous solution, prepared beforehand, of from 20 to 80°C for 2 hours, followed by cooling to 25°C. The assay is performed 24 hours after this preparation on the supernatant obtained after centrifuging the suspension to remove all crystals, or on the pellet in the case of solubilization of oily molecules, the centrifugation resulting in creaming of the portion that is not encapsulated in the micelles.
In order for the gain in solubility to be satisfactory, the solubility in the aqueous phase of the lipophilic compound encapsulated in the block copolymer must be at least 15 times greater than the "natural" solubility of the lipophilic compound in water.
Examples of lipophilic compounds that may be mentioned include emollients, anti-inflammatory agents, antibacterial agents, antifungal agents, antiviral agents, anti-seborrhoeic agents, antiacne agents, keratolytic agents, antihistamines, anesthetics, cicatrizing agents, pigmentation modifiers, tanning accelerators, artificial tanning agents, liporegulators, anti-ageing and anti-wrinkle agents, refreshing agents, vascular protectors, insect repellants, deodorants, antidandruff agents, agents for preventing hair loss, essential oils, which may be chosen especially from eucalyptus oil, lavandin oil, lavender oil, vetiver oil, Litsea cubeba oil, lemon oil, sandalwood oil, rosemary oil, camomile oil, savory oil, nutmeg oil, cinnamon oil, hyssop oil, caraway oil, orange oil, geraniol oil and cade oil, fragrances, sunscreens, antioxidants, free-radical scavengers and moisturizers.
Examples of lipophilic compounds that may also be mentioned include vitamins such as vitamin A (retinol) or esters thereof, vitamin E or esters thereof such as tocopheryl acetate, vitamin D or derivatives thereof and vitamin F or derivatives thereof; carotenes such as β-carotene and derivatives thereof such as lycopene, and salicylic acid derivatives, especially those described in documents FR-A-2 581 542, EP-A-378 936 and EP-A-570 230.

The salicylic acid derivatives may be chosen especially from the derivatives of formula 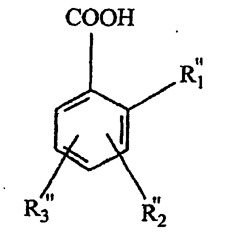 in which:
R"₁ represents a hydroxyl radical or an ester of formula -O-CO-R"₄ in which R"₄ is a saturated or unsaturated aliphatic radical containing from 1 to 26 carbon atoms and preferably from 1 to 18 carbon atoms, or an amine or thiol function optionally substituted with an alkyl radical containing from 1 to 18 carbon atoms and preferably from 1 to 12 carbon atoms,
R"₂ and R"₃, independently of each other, are in one of the positions 3, 4, 5 or 6 on the benzene nucleus and represent, independently of each other, a hydrogen atom or a radical: -(O)ₙ-(CO)ₘ-R"₅ in which n and m, independently of each other, are each an integer equal to 0 or 1, on condition that
R"₂ and R"₃ are not simultaneously hydrogen atoms; and R"₅ represents a hydrogen, a saturated, linear, branched or cyclized aliphatic radical containing from 1 to 18 carbon atoms, an unsaturated radical containing from 3 to 18 carbon atoms, bearing one to nine conjugated or nonconjugated double bonds, the radicals possibly being substituted with at least one substituent chosen from halogen atoms (fluorine, chlorine, bromine or iodine), trifluoromethyl radicals, hydroxyl radicals in free form or esterified with an acid containing from 1 to 6 carbon atoms, or carboxyl radicals in free form or esterified with a lower alcohol containing from 1 to 6 carbon atoms, or an aromatic radical containing from 6 to 10 carbon atoms.

Preferably, the salicylic acid derivative is such that R"₅ represents a saturated aliphatic radical containing from 3 to 15 carbon atoms.

Preferably, the salicylic acid derivative is such that R"₁ represents a hydroxyl radical.

Preferably, the salicylic acid derivative is such that R"₅ is in position 5 of the benzene nucleus and R"₂ represents a hydrogen atom.

According to one preferred embodiment of the invention, the salicylic acid derivative is chosen from the 5-n-octanoylsalicylic, 5-n-decanoylsalicylic, 5-n-dodecanoylsalicylic, 5-n-octylsalicylic, 5-n-heptyloxysalicylic, 4-n-heptyloxysalicylic, 5-tert-octylsalicylic, 3-tert-butyl-5-methylsalicylic, 3-tert-butyl-6-methylsalicylic, 3,5-diisopropylsalicylic, 5-butoxysalicylic, 5-octyloxysalicylic, 5-propanoylsalicylic, 5-n-hexadecanoylsalicylic, 5-n-oleoylsalicylic and 5-benzoylsalicylic derivatives, monovalent and divalent salts thereof, and mixtures thereof.

When it is a sunscreen, the lipophilic compound may be chosen from anthranilates; cinnamic derivatives; dibenzoylmethane derivatives; salicylic derivatives; camphor derivatives; triazine derivatives, preferably 1,3,5-triazine derivatives such as those described in patent applications US 4 367 390, US 4 724 137, EP 863 145, EP 517104, EP 570 838, EP 796 851, EP 775 698, EP 878 469, EP 933 376, EP 507 691, EP 507 692, EP 790 243 and EP 944 624; benzophenone derivatives; β,β'-diphenylacrylate derivatives; benzotriazole derivatives; benzalmalonate derivatives; benzimidazole derivatives; imidazolines; bis-benzazolyl derivatives as described in patents EP 669 323 and US 2 463 264; p-aminobenzoic acid (PABA) derivatives; methylene-bis(hydroxyphenylbenzotriazole) derivatives as described in patent applications US 5 237 071, US 5 166 355, GB 2 303 546, DE 197 26 184 and EP 893 119; screening polymers and screening silicones such as those described especially in patent application WO 93/04665; dimers derived from α-alkylstyrene, such as those described in patent application DE 198 55 649; 4,4-diarylbutadienes as described in patent applications EP 0 967 200 and DE 197 55 649, and mixtures thereof.

One preferential 1,3,5-triazine is 2,4,6-tris[p-(2'-ethylhexyl-1'-oxycarbonyl)anilino]-1,3,5-triazine, which is a UV-B-active screening agent that is known per se, which is in a solid form and which is sold especially under the trade name "Uvinul T150" by the company BASF. This product corresponds to the following formula: 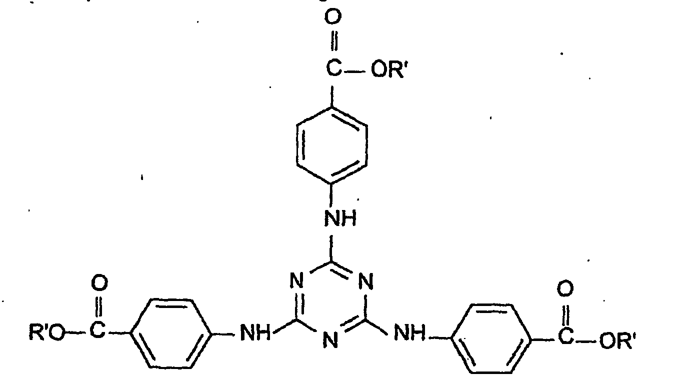 in which R' denotes a 2-ethylhexyl radical.

Other 1,3,5-triazine derivatives used according to the invention that are particularly preferred are 2-[(p-(tert-butylamido)anilino]-4,6-bis[(p-(2'-ethylhexyl-1'-oxycarbonyl)anilino]-1,3,5-triazine, 2,4-bis{[4-2-ethylhexyloxy]-2-hydroxy]phenyl}-6-(4-methoxyphenyl)-1,3,5-triazine, 2,4,6-tris(diisobutyl 4'-aminobenzalmalonate)-s-triazine, and mixtures thereof.

Among the dibenzoylmethane derivatives, it is most particularly preferred to use 4-(tert-butyl)-4'-methoxydibenzoylmethane, or butylmethoxydibenzoylmethane, sold especially under the trade name "Parsol^{®} 1789" by the company Hoffmann Laroche.

According to the present invention, the lipophilic compounds may also be chosen from:
(1) dehydroepiandrosterone (DHEA) and its precursors and chemical and biological derivatives, with the exception of cholesterol and its esters and plant sterols such as phytosterols and sitosterols, and esters thereof; dehydroepiandrosterone is a natural steroid produced essentially by the adrenal glands, corresponding to the formula 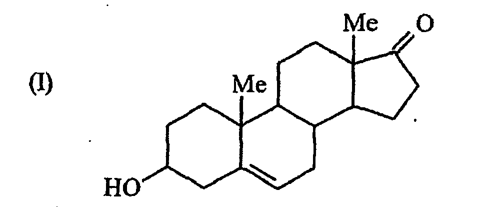
   It is known for its anti-ageing properties associated with its capacity to promote epidermal keratinization (JP-07 196 467) and to combat osteoporosis (US 5 824 671), or in the treatment of dry skin, on account of its ability to increase the endogenous production and secretion of sebum and to reinforce the skin's barrier effect (US 4 496 556). It has also been proposed to use DHEA sulphate against alopecia (JP-60 142 908) and to treat various signs of ageing such as wrinkles, loss of radiance of the skin and slackening of the skin (EP-0 723 775).
   The DHEA that may be used according to the invention is available, for example, from the companies Sigma and Akzo Nobel.
   The expression "DHEA precursor" means the immediate biological precursors thereof and also the chemical precursors thereof. Examples of biological precursors are pregnenolone, 17α-hydroxypregnenolone, 5-androstenediol, 17α-hydroxypregnenolone sulphate and 5-androstenediol sulphate. Examples of chemical precursors are sapogenins such as diosgenin (spirost-5-ene-3-beta-ol), hecogenin, smilagenin and sarsapo-genin, and also natural extracts containing them, in particular fenugreek and extracts of Dioscorea plants such as wild yam root.
   The expression "DHEA derivatives" means both the metabolic derivatives thereof and the chemical derivatives thereof. Metabolic derivatives which may be mentioned in particular include 7α-hydroxy-DHEA, 7-keto-DHEA, 5-androstene-3β,17β-diol (or adiol), 5-androstene-3β,17β-diol sulphate and 4-androstene-3,17-dione, although this list is not limiting. Chemical derivatives that may be mentioned in particular include salts, in particular water-soluble salts such as DHEA sulphate, esters such as the hydroxycarboxylic acid esters of DHEA described in US 5 736 537 or other esters such as DHEA salicylate, acetate, valerate and enanthate;
(2) pentacyclic triterpene acids such as ursolic acid and oleanolic acid. They are present in plants such as rosemary. They are frequently used in pharmaceutical compositions for their numerous therapeutic properties, and especially for their anti-inflammatory, hepato-protective, diuretic, analgesic and antimicrobial properties, their inhibitory properties on certain enzymatic activities, and their antitumour properties. In the cosmetic field, ursolic acid is described, for example, as a constituent of an antiperspirant composition (FR A 2 541 895) and as an inhibitor of the activity of tyrosinase, a key enzyme in melanin synthesis (JP-58/57307).
(3) hydroxystilbenes, which are compounds corresponding to the general formula: 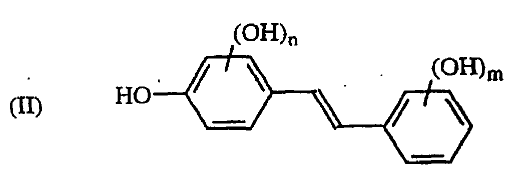 in which n is an integer between 1 and 4 inclusive and m is an integer between 1 and 5 inclusive. This formula includes the cis and *trans* compounds. According to the present invention, the term "hydroxystilbene" also covers the hydroxyalkyl derivatives of the compounds of formula (II). Hydroxystilbenes are compounds that are found in the natural state in plants of the spermatophyte class and in particular in vine. In the cosmetic field, hydroxystilbenes are used, inter alia, as depigmenting agents (JP-87-192 040) or anti-ageing agents (FR-2 777 186).
   Among the hydroxystilbenes that may be mentioned are mono-, di-, tri-, tetra-, penta-, hexa-, hepta-, octa- and nonahydroxystilbenes, or hydroxyalkyl derivatives thereof. According to the invention, the hydroxystilbenes may be used alone or as mixtures of any nature and may be of natural or synthetic origin. The hydroxystilbenes that may be used according to the invention are chosen from:
   4'-hydroxystilbene,
   2',4'-dihydroxystilbene,
   3',4'-dihydroxystilbene,
   4,4'-dihydroxystilbene,
   2',4',4-trihydroxystilbene,
   3',4',4-trihydroxystiibene,
   2,4,4'-trihydroxystilbene,
   3,4,4'-trihydroxystilbene,
   3,5,4'-trihydroxystilbene,
   2',3,4-trihydroxystilbene,
   2,3',4-trihydroxystilbene,
   2',2,4'-trihydroxystilbene,
   2,4,4',5'-tetrahydroxystilbene,
   2',3,4',5-tetrahydroxystilbene,
   2,2',4,4'-tetrahydroxystilbene,
   3,3',4',5-tetrahydroxystilbene,
   2,3',4,4'-tetrahydroxystilbene,
   3,3',4,4'-tetrahydroxystilbene,
   3;3',4',5,5'-pentahydroxystilbene,
   2,2',4,4',6-pentahydroxystilbene,
   2,3',4,4',6-pentahydroxystilbene,
   2,2',4,4',6,6'-hexahydroxystilbene.

   Among these compounds, resveratrol (3,5,4'-trihydroxystilbene) is particularly preferred since it is naturally present in the skin of grape seeds and in wine. In this regard, the review by Soleas and collaborators (Clinical Biochemistry, vol. 30, No. 2, pages 91 - 113, 1997), which perfectly summarizes the state of knowledge regarding this compound and hydroxystilbenes, may be consulted.
(4) isoflavonoids, a sub-class of flavonoids, which are formed from a 3-phenylchroman skeleton that is oxidized to a greater or lesser extent and that may bear various substituents. The term "isoflavonoid" covers several classes of compounds, among which mention may be made of isoflavones, isoflavanones, rotenoids, pterocarpans, isoflavans, isoflavan-3-enes, 3-arylcoumarins, 3-aryl-4-hydroxycoumarins, coumestanes, coumarono-chromones or 2-arylbenzofurans. A full review of isoflavonoids, their sources and methods of analysis has been published in "The Flavonoids", Harbone editor (1988), chapter 5 entitled "Isoflavonoids" by P.M. Dewick, pages 125-157.
   The isoflavonoids used according to the invention have a solubility in water at room temperature (25°C) of less than 0.01 % and may be of natural origin, that is to say extracts of an element of natural origin, usually a plant, or may have been obtained by chemical synthesis, isoflavonoids of natural origin are preferred.
   An example of an isoflavonoid of natural origin that may be mentioned is genistin.
   A preferred sub-class of isoflavonoids is that of isoflavones, covering both the aglycone forms (daidzein, genistein and glycitein) and the glycosyl forms (daidzin, genistin, glycitin).
   Processes for preparing isoflavones are described in particular in patents and patent applications WO 95/10530, WO 95/10512, US 5 679 806, US 5 554 519, EP 812 837 and WO 97/26269.
   Isoflavones are known in particular as antioxidants, for their free-radical-scavenging and depigmenting properties, and also for their capacity to inhibit the activity of sebaceous glands (DE-44 32 947). They have also been described as agents capable of preventing signs of ageing of the skin (JP 1-96106).
(5) aminophenol derivatives of formula 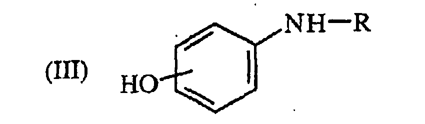
in which R is a radical corresponding to one of the formulae (i), (ii) and (iii) below
(i) -CO-NR₁R₂
(ii) -CO-O-R₃
(iii) -SO₂-R₃
in which R₁ represents a hydrogen atom or an optionally hydroxylated, saturated or unsaturated, linear or branched C₁₋₆ alkyl radical,
R₂ represents a hydrogen atom or a radical chosen from optionally hydroxylated, saturated or unsaturated, linear, cyclic or branched C₁₂ to C₃₀ alkyl radicals, and
R₃ represents a radical chosen from saturated or unsaturated, linear, branched or cyclic C₁₂ to C₃₀ alkyl radicals, including fused polycyclic radicals, which are optionally hydroxylated. ,

Examples of lipophilic compounds that are preferred according to the present invention which may be mentioned include dehydroepiandrosterone (DHEA), DHEA sulphate, 7α-hydroxy-DHEA, 7-keto-DHEA, prednisolone, prednisone, progesterone, pregnenolone, testosterone, diosgenin, hecogenin, ursolic acid, oleanolic acid, resveratrol (= 3,5,4'-trihydroxy-stilbene) and N-cholesteryloxycarbonyl-4-aminophenol, and isoflavonoids whose solubility in water at room temperature (25°C) is less than 0.01%.

A subject of the invention is also a cosmetic composition comprising at least one aqueous phase, at least one lipophilic compound and an effective amount of at least one block amphiphilic copolymer as above defined.

The lipophilic compound may be present in the composition in weight contents of between 0.001% and 10% of the total weight of the composition.

The method for implementing the block copolymer(s) and the lipophilic compound(s) to be combined will be chosen by a person skilled in the art as a function of the chemical nature of the polymer. Examples that will be mentioned include nanoprecipitation via a water-miscible solvent, dialysis or direct hydration of the pre-combined block copolymer/lipophilic compound mixture. Nanoprecipitation consists in co-dissolving, in a water-miscible organic solvent with a boiling point below that of water, the block copolymer(s) and the lipophilic compound to be encapsulated and in introducing this organic solution more or less quickly into an aqueous phase, with stirring (for example using a magnetic bar, paddles or a turbomixer). The micelles or particles form instantaneously. The solvent is then evaporated off.
The dialysis method consists in co-dissolving, in a water-miscible to sparingly water-miscible organic solvent with a boiling point higher than that of water, the block copolymer(s) and the lipophilic compound to be encapsulated. This solution is introduced into a dialysis bag and dialysed against water. The dialysis water is regularly renewed, until the solvent has been completely dialysed. The same method may be envisaged with surfactants, for instance octyl glucoside, instead of solvent. It is a concentrated aqueous solution of surfactants that dissolves the copolymer(s) and the lipophilic compound to give a clear solution. This solution is then dialysed until the surfactant is completely removed.
Another method, known as the Bangham method, which is commonly used for making liposomes, may be used. This consists in dissolving, in an organic solvent, the block copolymer(s) and the lipophilic compound to be encapsulated, and then evaporating off the solvent so as to obtain a uniform mixture of the block copolymer(s) and of the lipophilic compound to be encapsulated. A rotary evaporator may be used in the laboratory, an atomizer may be used industrially, or any other method for evaporating a solvent may be used. This mixture may then be hydrated directly, with stirring, using an aqueous solution.
In general, any method known to those skilled in the art for synthesizing liposomes and nanoparticles may be used.
According to one embodiment of the invention, the block amphiphilic copolymer is the sole solvent for the lipophilic compound in the composition.

The compositions according to the invention may also contain pigments or nanopigments (mean size of the primary particles: generally between 5 nm and 100 nm and preferably between 10 and 50 nm) of coated or uncoated metal oxides, for instance nanopigments of titanium oxide (amorphous or crystallized in rutile and/or anatase form), iron oxide, zinc oxide, zirconium oxide or cerium oxide, which are photoprotective agents that are well known per se, acting by physically blocking (reflection and/or scattering) UV radiation. Standard coating agents are, moreover, alumina and/or aluminium stearate. Such coated or uncoated metal oxide nanopigments are described in particular in patent applications EP-A-0 518 772 and EP-A-0 518 773.
The compositions in accordance with the present invention may also contain standard cosmetic adjuvants chosen especially from fatty substances, organic solvents, ionic or nonionic thickeners, softeners, free-radical scavengers, opacifiers, stabilizers, emollients, silicones, α-hydroxy acids, antifoams, moisturizers, vitamins, preserving agents, surfactants, fillers, sequestering agents, polymers, propellants, acidifying or basifying agents, dyes or any other ingredient usually used in cosmetics and/or dermatology, in particular to manufacture antisun compositions in the form of emulsions.
The fatty substances may consist of an oil or a wax or mixtures thereof. The term "oil" means a compound that is liquid at room temperature. The term "wax" means a compound that is solid or substantially solid at room temperature, and which has a melting point generally above 35°C.
Oils that may be mentioned include mineral oils (vaseline); plant oils (sweet almond oil, macadamia oil, blackcurrant seed oil or jojoba oil); synthetic oils, for instance perhydrosqualene, fatty alcohols, fatty acids or fatty esters (for instance the C₁₂-C₁₅ alcohol benzoates sold under the name "Finsolv TN" by the company Finetex, octyl palmitate, isopropyl lanolate, triglycerides including those of capric/caprylic acids), ethoxylated or propoxylated fatty esters and ethers; silicone oils (cyclomethicone preferably containing 4 or 5 silicon atoms, and polydimethylsiloxane or PDMS) or fluoro oils, and polyalkylenes.
Waxy compounds that may be mentioned include paraffin, carnauba wax, beeswax and hydrogenated castor oil.
Among the organic solvents that may be mentioned are lower alcohols and polyols.
The thickeners may be chosen especially from crosslinked polyacrylic acids, modified or unmodified guar gums and celluloses, such as hydroxypropyl guar gum, methylhydroxyethylcellulose or hydroxypropylmethylcellulose, and silicone gums, for instance a polydimethylsiloxane derivative.
The compositions of the invention may be prepared according to the techniques that are well known to those skilled in the art, in particular those intended for preparing emulsions of the oil-in-water or water-in-oil type.
These compositions may be in particular in the form of a dispersion, especially an aqueous dispersion, a simple or complex emulsion (O/W, W/O, O/W/O or W/O/W emulsion) such as a cream, a milk or a cream-gel, a powder or a solid tube, and may optionally be packaged as an aerosol and may be in the form of a mousse or a spray.
When it is an emulsion, the aqueous phase of the said emulsion may comprise a nonionic vesicular dispersion prepared according to known processes (Bangham, Standish and Watkins, J. Mol. Biol. 13, 238 (1965), FR 2315991 and FR 2416008)..

The cosmetic compositions according to the invention may be used as compositions for protecting the human epidermis or the hair against ultraviolet rays, as antisun compositions or as makeup products.
When the cosmetic compositions according to the invention are used for protecting the human epidermis against UV rays or for antisun compositions, they may be in the form of a suspension or a dispersion in fatty substances, in the form of a nonionic vesicular dispersion or in the form of an emulsion, preferably of oil-in-water type, such as a cream or a milk, or in the form of an ointment, a gel, a solid tube, a stick, an aerosol mousse or a spray.
When the cosmetic compositions according to the invention are used for protecting the hair, they may be in the form of a shampoo, a lotion, a gel, an emulsion or a nonionic vesicular dispersion. They may constitute, for example, a rinse-out composition, to be applied before or after shampooing, before or after dyeing or bleaching, or before, during or after permanent-waving or relaxing the hair, a styling or treating lotion or gel, a blow-drying or hairsetting lotion or gel, or a composition for permanent-waving, relaxing, dyeing or bleaching the hair.
When the cosmetic compositions according to the invention are used as makeup products for the eyelashes, the eyebrows or the skin, such as an epidermal treatment cream, a foundation, a tube of lipstick, an eyeshadow, a makeup rouge, a mascara or an eyeliner, they may be in solid or pasty, anhydrous or aqueous form, for instance oil-in-water or water-in-oil emulsions, nonionic vesicular dispersions or suspensions.
The examples that follow are intended to illustrate the invention.

### Example 1

The block copolymer is dissolved in dichloromethane with excess DHEA. The solvent is evaporated off under reduced pressure using a rotary evaporator. The film formed is then hydrated with distilled water, with stirring, at a temperature of between 20 and 80°C, for 2 hours. The polymer content in the water is of the order of one percent. 24 hours later, this suspension is centrifuged so as to separate out the DHEA not encapsulated in the polymer micelles.
The supernatant is taken up and then assayed by HPLC.

| Chemical nature | Solubility | Solubility gain | DHEA/ polymer mass ratio | MW | A/B or A/B/A |
|---|---|---|---|---|---|
| Water | 0.001 | | | | |
| PS-POE | 0.061 | 61 | 1.22% | 2000 | 1000/1000 |
| PS-POE | 0.036 | 36 | 0.72% | 4000 | 1000/3000 |
| Poly(methyl methacrylate)/ POE | 0.055 | 55 | 1.10% | 2000 | 1000/1000 |
| Poly(methyl methacrylate)/ POE | 0.023 | 23 | 0.46% | 4000 | 1000/3000 |
| Poly(butyl methacrylate)/ POE | 0.052 | 25 | 1.04% | 2000 | 1000/1000 |
| Poly(butyl methacrylate)/ POE | 0.034 | 34 | 0.68% | 4000 | 1000/3000 |
| POE-POB-POE | 0.031 | 31 | 0.62% | 9000 | 3500/2000/ 3500 |
| PS-POE | 0.01 | 10 | 0.20% | 10600 | 3600/7000 |
| PS-POE | 0.06 | 60 | 1.20% | 9000 | 3900/5100 |
| PS-POE/PS-POE (90/10) | 0.02 | 20 | 0.40% | | 3600/7000/ 12200/239 00 |
| PS-POE/PS-POE (90/10) | 0.08 | 80 | 1.60% | | 3900/5100/ 12200/239 00 |
| POE-POP-POE (comparative) | 0.005 | 5 | 0.10% | 14 600 | 128/54/128 |
| POE-POP-POE (comparative) | 0.002 | 2 | 0.04% | 8400 | 75/30/75 |
| POE-POP-POE (comparative) | 0.003 | 1 | 0.06% | 2200 | |
| POE-POP-POE (comparative) | 0.002 | 2 | 0.04% | 4700 | 46/16/46 |

| | | | | | |
|---|---|---|---|---|---|
| PS: polystyrene POE: polyoxyethylene POP: polyoxypropylene POB: polyoxybutylene | | | | | |

The solubility gain is calculated relative to the "natural" solubility of the lipophilic compound in water.
It is found that the POE-POP-POE block copolymers do not make it possible to dissolve the DHEA sufficiently (less than fifteen times).
On the other hand, the polymers according to the invention, for instance PS-POE, methyl methacrylate-POE, butyl methacrylate-POE or POE-POB-POE, make it possible to improve the solubility of DHEA in water, up to 80 times.

### Example 2

The block polymer is dissolved in dichloromethane with excess Parsol^{®} 1789. The solvent is evaporated off under reduced pressure using a rotary evaporator. The film formed is then hydrated with distilled water, with stirring, at a temperature of between 20 and 80°C, for 2 hours. The polymer content in the water is of the order of one percent. 24 hours later, this suspension is centrifuged in order to separate out the Parsol^{®} 1789 that has not been encapsulated in the polymer micelles. The supernatant, which should not contain any crystals that may be identified using an optical microscope in polarized light, is taken up and then assayed by HPLC.

| Chemical nature | Solubility mg/ml | Solubility gain | Parsol 1789/ polymer mass ratio | A/B or A/B/A |
|---|---|---|---|---|
| Water | 0.001 | | | |
| PS-POE | 0.686 | 686 | 3.43% | 1000/1000 |
| PS-POE | 0.34 | 340 | 1.70% | 1000/3000 |
| Methyl methacrylate/POE | 0.862 | 862 | 4.31 % | 1000/1000 |
| Methyl methacrylate/POE | 0.43 | 430 | 2.15% | 1000/3000 |
| Butyl methacrylate/POE | 1.008 | 1008 | 5.04% | 1000/1000 |
| Butyl methacrylate/POE | 0.475 | 475 | 2.37% | 1000/3000 |
| POE-POB-POE | 0.230 | 230 | 1.18% | 3500/2000/ 3500 |
| PDMS/OE/OP | 0.171 | 171 | 0.85% | PDMS/OE/ OP |
| PDMS/OE/OP | 0.186 | 186 | 0.93% | PDMS/OE/ OP |
| PS-POE | 0.171 | 171 | 0.85% | 3600/7000 - |
| PS-POE | 0.185 | 185 | 0.92% | 3900/5100 |
| PS-POE/PS-POE (90/10) | 0.53 | 530 | 2.65% | 3600/7000/ 12200/2390 0 |
| PS-POE/PS-POE (90/20) | 0.453 | 453 | 2.26% | 3900/5100/ 12200/2390 0 |
| PS-POE | 0.529 | 529 | 2.64% | 2300/3100 |
| PE-POE | 0.164 | 164 | 0.82% | 5000/5900 |
| PE-POE | 0.124 | 124 | 0.62% | 4800/4800 |
| POE-POP-POE (comparative) | 0.006 | 6 | 0.03% | 75/30/75 |

| | | | | |
|---|---|---|---|---|
| PS: polystyrene POE: polyoxyethylene POP: polyoxypropylene POB: polyoxybutylen | | | | |

The solubility gain is calculated relative to the "natural" solubility of the lipophilic compound in water.
Surprisingly, in this case also, the block copolymers according to the invention dissolve the lipophilic compound better than do the POE-POP-POE block copolymers.

### Example 3 : Oil-in-water emulsion

| *Oily phase:* | |
|---|---|
| Diglyceryl monostearate | 2.0 % |
| PEG-20 stearate | 1.5' % |
| Disodium N-stearoyl-L-glutamic acid | 0.5 % |
| (Acyl glutamate HS 21 from Ajinomoto) | |
| Liquid petroleum jelly | 3 % |
| Petroleum jelly | 1 % |
| Stearyl heptanoate | 3 % |
| Apricot kernel oil | 5 % |
| Hydrogenated polyisobutene | 5 % |
| Isocetyl palmitate | 2 % |
| Volatile silicone | 5 % |
| Vitamin E | 0.5 % |
| Preserving agent | 0.3 % |
| | |
| Aqueous phase 1 | |
| Glycerol | 5 % |
| Preserving agents | 1 % |
| Distilled water qs | 100 % |
| | |
| Aqueous phase 2 | |
| Carbomer | 0.4 % |
| Distilled water | 15 % |
| Preserving agents | 0.1 % |
| Triethanolamine | 0.4 % |
| | |
| Aqueous phase 3 | |
| Aqueous suspension of ME 10-10 | |
| (methyl methacrylate/POE 1000/1000) at 20% in water, containing 8% Parsol^{®} 1789 | 10 % |

| | |
|---|---|
| Procedure: The aqueous phase 1 is introduced at 60°C into the oily phase at 60°C, with very vigorous stirring. The temperature and the stirring are maintained for 30 minutes. The suspension is then cooled to room temperature. The aqueous phase 2 is then dispersed using a non-shear disperser. The aqueous phase 3 (micellar suspension) is then introduced with gentle stirring. | |

### Example 4 : Oil-in-water emulsion

| | |
|---|---|
| *Oily phase:* | |
| Diglyceryl monostearate | 2.0 % |
| PEG-20 stearate | 1.5 % |
| Disodium N-stearoyl-L-glutamic acid | 0.5 % |
| (Acyl glutamate HS 21 from Ajinomoto) | |
| Liquid petroleum jelly | 3 % |
| Petroleum jelly | 1 % |
| Stearyl heptanoate | 3 % |
| Apricot kernel oil | 5 % |
| Hydrogenated polyisobutene | 5 % |
| Isocetyl palmitate | 2 % |
| Volatile silicone | 5 % |
| Vitamin E | 0.5 % |
| Preserving agent | 0.3 % |
| | |
| Aqueous phase 1 | |
| Glycerol | 5 % |
| Preserving agents | 1 % |
| Distilled water qs | 100 % |
| | |
| Aqueous phase 2 | |
| Carbomer | 0.4 % |
| Distilled water | 15 % |
| Preserving agents | 0.1 % |
| Triethanolamine | 0.4 % |
| | |
| Aqueous phase 3 | |
| Aqueous suspension of PS-POE (1000-1000) at 20% in water, containing 1% DHEA relative to the polymer | |
| | 10 % |

| | |
|---|---|
| Procedure: The aqueous phase 1 is introduced at 60°C into the oily phase at 60°C, with very vigorous stirring. The temperature and the stirring are maintained for 30 minutes. The suspension is then cooled to room temperature. The aqueous phase 2 is then dispersed using a non-shear disperser. The aqueous phase 3 (micellar suspension) is then introduced with gentle stirring. | |

### Example 5

An essential oil of lavender is dissolved, forming a micellar suspension of butyl methacrylate-POE (1000-1000)

| | |
|---|---|
| Aqueous phase 1 | |
| Butyl methacrylate-POE (1000-1000) | 10 % |
| Essential oil of lavender | 0.15 % |
| Distilled water | 80 % |
| | |
| Aqueous phase 2 | |
| Glycerol | 3 % |
| Preserving agents | 0.3 % |
| Distilled water qs | 100 % |

| | |
|---|---|
| Procedure: The aqueous phase 1 is heated to 60°C and is then mixed with stirring with the aqueous phase 2, which has itself been preheated to 60°C. The temperature and the stirring are maintained for 30 minutes. The composition is then cooled to room temperature. | |

## Claims

1. Process for dissolving at least one lipophilic compound in an aqueous phase, **characterized in that** it comprises the step of associating the said lipophilic compound with an effective amount of at least one amphiphilic block copolymer comprising at least one ionic and/or at least one nonionic hydrophilic polymer block, and at least one hydrophobic polymer block obtained from at least one hydrophobic monomer chosen from :
- styrene and its derivatives such as 4-butylstyrene,
- vinyl acetate of formula CH₂=CH-OCOCH₃,
- vinyl ethers of formula CH₂=CHOR in which R is a linear or branched, saturated or unsaturated hydrocarbon-based radical containing from 1 to 6 carbon atoms,
- acrylonitrile,
- vinyl chloride and vinylidene chloride,
- caprolactam,
- alkenes such as ethylene, propylene, butylene and butadiene,
- alkylene oxides containing at least 4 carbon atoms and preferably from 4 to 6 carbon atoms,
- silicone derivatives producing, after polymerization, silicone polymers such as polydimethylsiloxane,
- hydrophobic vinyl monomers of formula (A) below: 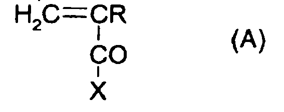
in which:
- R is chosen from H, -CH₃, -C₂H₅ and -C₃H₇,
- X is chosen from:
- alkyl oxides of -OR' type in which R' is a linear, cyclic or branched, saturated or unsaturated hydrocarbon-based radical containing from 1 to 22 carbon atoms, optionally substituted with a halogen atom (iodine, bromine, chlorine or fluorine); a sulphonic group (-SO₃⁻), a sulphate group (-SO₄⁻), a phosphate group (-PO₄H₂⁻); a hydroxyl group (-OH); a primary amine group (-NH₂); a secondary amine group (-NHR₁), a tertiary amine group (-NR₁R₂) or a quaternary amine group (-N⁺R₁R₂R₃) with R₁, R₂ and R₃ being, independently of each other, a linear or branched, saturated or unsaturated hydrocarbon-based radical containing from 1 to 22 carbon atoms, with the proviso that the sum of the carbon atoms of R' + R₁ + R₂ + R₃ does not exceed 22; R' may also be a perfluoroalkyl radical, preferably containing from 1 to 18 carbon atoms;
- NH₂, -NHR' and -NR'R" groups in which R' and R" are, independently of each other, linear, cyclic or branched, saturated or unsaturated hydrocarbon-based radicals containing from 1 to 22 carbon atoms, with the proviso that the total number of carbon atoms of R' + R" does not exceed 22, R' and R" optionally being substituted with a halogen atom (iodine, bromine, chlorine or fluorine); a hydroxyl group (-OH); a sulphonic group (-SO₃⁻); a sulphate group (-SO₄⁻); a phosphate group (-PO₄H₂⁻); a primary amine group (-NH₂); a secondary amine group (-NHR₁), a tertiary amine group (-NR₁R₂) and/or a quaternary amine group (-N⁺R₁R₂R₃) with R₁, R₂ and R₃ being, independently of each other, a linear or branched, saturated or unsaturated hydrocarbon-based radical containing from 1 to 22 carbon atoms, with the proviso that the sum of the carbon atoms of R' + R" + R₁ + R₂ + R₃ does not exceed 22; R' and R" may also be perfluoroalkyl radicals, preferably containing from 1 to 18 carbon atoms.

2. Process according to claim 1, **characterized in that** the ionic hydrophilic polymer block is polyethyleneimine or is obtained from water-soluble monomers or salts thereof, chosen from
- (meth)acrylic acid,
- acrylamido-2-methylpropanesulphonic (AMPS) acid,
- styrenesulphonic acid,
- vinylsulphonic acid and (meth)allylsulphonic acid,
- vinylphosphonic acid,
- maleic anhydride,
- itaconic acid,
- dimethyldiallylammonium chloride,
- quaternized dimethylaminoethyl methacrylate (DMAEMA),
- (meth)acrylamidopropyltrimethylammonium chloride (APTAC and MAPTAC),
- methylvinylimidazolium chloride,
- hydrophilic vinyl monomers of formula (A) below: 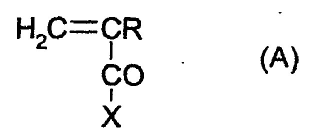
in which:
- R is chosen from H, -CH₃, -C₂H₅ and -C₃H₇,
- X is chosen from:
- alkyl oxides of -OR' type in which R' is a linear, cyclic or branched, saturated or unsaturated hydrocarbon-based radical containing from 1 to 6 carbon atoms, substituted with at least one sulphonic group (-SO₃⁻) and/or sulphate group (-SO₄⁻) and/or phosphate group (-PO₄H₂⁻) and/or quaternary amine group (-N⁺R₁R₂R₃) with R₁, R₂ and R₃ being, independently of each other, a linear or branched, saturated or unsaturated hydrocarbon-based radical containing from 1 to 6 carbon atoms, with the proviso that the sum of the atoms of R' + R₁ + R₂ + R₃ does not exceed 6; the radical R' being optionally substituted with a halogen, atom (iodine, bromine, chlorine or fluorine); a hydroxyl group (-OH); a primary amine group (-NH₂); a secondary amine group (-NHR₁) or a tertiary amine group (-NR₁R₂) with R₁, R₂ being, independently of each other, a linear or branched, saturated or unsaturated hydrocarbon-based radical containing from 1 to 6 carbon atoms, with the proviso that the sum of the carbon atoms of R' + R₁ + R₂ does not exceed 6;
- -NH₂, -NHR' and -NR'R" groups in which R' and R" are, independently of each other, linear, cyclic or branched, saturated or unsaturated hydrocarbon-based radicals containing from 1 to 6 carbon atoms, with the proviso that the total number of carbon atoms of R' + R" does not exceed 6, R' and/or R" optionally being substituted with at least one sulphonic group (-SO₃⁻) and/or sulphate group (-SO₄⁻) and/or phosphate group (-PO₄H₂⁻) and/or quaternary amine group (-N⁺R₁R₂R₃) with R₁, R₂ and R₃ being, independently of each other, a linear or branched, saturated or unsaturated hydrocarbon-based radical containing from 1 to 6 carbon atoms, with the proviso that the sum of the atoms of R' + R₁ + R₂ + R₃ does not exceed 6; the radicals R' and/or R" being optionally substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁, R₂ being, independently of each other, a linear or branched, saturated or unsaturated hydrocarbon-based radical containing from 1 to 6 carbon atoms, with the proviso that the sum of the carbon atoms of R' + R" + R₁ + R₂ does not exceed 6.

3. Process according to any one of the preceding claims, **characterized in that** the ionic hydrophilic block is chosen from (meth)acrylic acid.

4. Process according to any one of the preceding claims, **characterized in that** the nonionic hydrophilic polymer block is of polyoxyalkylenated type, for instance polyoxyethylene, or is polyvinylpyrrolidone (PVP), or is obtained from water-soluble monomers chosen from
- (meth)acrylamide,
- N-vinylacetamide and N-methyl-N-vinylacetamide,
- N-vinylformamide and N-methyl-N-vinylformamide,
- N-vinyllactams comprising a cyclic alkyl group containing from 4 to 9 carbon atoms, such as N-vinylpyrrolidone, N-butyrolactam and N-vinylcaprolactam,
- vinyl alcohol of formula CH₂=CHOH,
- glycidyl (meth)acrylate,
- hydrophilic vinyl monomers of formula (A) below: 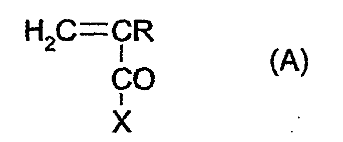
in which:
- R is chosen from H, -CH₃, -C₂H₅ and -C₃H₇,
- X is chosen from:
- alkyl oxides of -OR' type in which R' is a linear, cyclic or branched, saturated or unsaturated hydrocarbon-based radical containing from 1 to 6 carbon atoms, optionally substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁ and R₂ being, independently of each other, a linear or branched, saturated or unsaturated hydrocarbon-based radical containing from 1 to 6 carbon atoms, with the proviso that the sum of the carbon atoms of R' + R₁ + R₂ does not exceed 6;
- -NH₂, -NHR' and -NR'R" groups in which R' and R" are, independently of each other, linear, cyclic or branched, saturated or unsaturated hydrocarbon-based radicals containing from 1 to 6 carbon atoms, with the proviso that the total number of carbon atoms of R' + R" does not exceed 6, R' and R" optionally being substituted with a halogen atom (iodine, bromine, chlorine or fluorine); a hydroxyl group (-OH); a primary amine group (-NH₂); a secondary amine group (-NHR₁) or a tertiary amine group (-NR₁R₂) with R₁ and R₂ being, independently of each other, a linear or branched, saturated or unsaturated hydrocarbon-based radical containing from 1 to 6 carbon atoms, with the proviso that the sum of the carbon atoms of R' + R" + R₁ + R₂ does not exceed 6.

5. Process according to Claim 4, **characterized in that** the nonionic hydrophilic polymer block is chosen from polyethylene oxide and polyvinyl-pyrrolidone.

6. Process according to any one of the preceding claims, **characterized in that** the hydrophilic polymer block is nonionic.

7. Process according to any one of the preceding claims, **characterized in that** the hydrophobic polymer block is obtained from at least one hydrophobic monomer chosen from styrene, tert-butylstyrene, methyl methacrylate, ethyl (meth)acrylate, n-butyl (meth)acrylate, tert-butyl (meth)acrylate, cyclohexyl acrylate, isobornyl acrylate, 2-ethylhexyl acrylate, ethyl perfluorooctyl acrylate, trifluoromethyl (meth)acrylate, polybutylene oxide or polyoxybutylene (POB), butadiene, ethylene, propylene and butylene.

8. Process according to any one of the preceding claims, **characterized in that** the block copolymer is chosen from the following block copolymers:
- polystyrene/polyoxyethylene
- polymethyl methacrylate/polyoxyethylene
- polybutyl methacrylate/polyoxyethylene
- polyoxybutylene/polyoxyethylene
- polycaprolactone/polyoxyethylene
- polyethylene/polyoxyethylene
- polyoxyethylene/polyoxybutylene/polyoxyethylene.

9. Process according to any one of the preceding claims, **characterized in that** the molecular weight of the block copolymer is between 1 000 and 500 000, preferably from 2000 to 100 000.

10. Process according to any one of the preceding claims, **characterized in that** the weight ratio A/B between the hydrophilic block A and the hydrophobic block B ratio is between 1/100 and 50/1.

11. Process according to any one of the preceding claims, **characterized in that** the weight concentration ratio between the lipophilic compound and the amphiphilic block copolymer is between 0.005 and 0.5 and preferably between 0.005 and 0.2.

12. Process according to any one of the preceding claims, **characterized in that** the lipophilic compound is chosen from :
- emollients, anti-inflammatory agents, antibacterial agents, antifungal agents, antiviral agents, anti-seborrhoeic agents, antiacne agents, keratolytic agents, antihistamines, anaesthetics, cicatrizing agents, pigmentation modifiers, tanning accelerators, artificial tanning agents, liporegulators, anti-ageing and anti-wrinkle agents, refreshing agents, vascular protectors, insect repellants, deodorants, antidandruff agents, agents for preventing hair loss,
- essential oils, which may be chosen especially from eucalyptus oil, lavandin oil, lavender oil, vetiver oil, Litsea cubeba oil, lemon oil, sandalwood oil, rosemary oil, camomile oil, savory oil, nutmeg oil, cinnamon oil, hyssop oil, caraway oil, orange oil, geraniol oil and cade oil, fragrances,
- sunscreens, antioxidants, free-radical scavengers and moisturizers;
- vitamins such as vitamin A (retinol) or esters thereof, vitamin E or esters thereof such as tocopheryl acetate, vitamin D or derivatives thereof and vitamin F or derivatives thereof; carotenes such as β-carotene and derivatives thereof such as lycopene, and salicylic acid derivatives;
- dehydroepiandrosterone (DHEA) and its biological precursors and derivatives, with the exception of cholesterol and its esters and plant sterols such as phytosterols and sitosterols, and esters thereof,
- pentacyclic triterpene acids such as ursolic acid and oleanolic acid,
- hydroxystilbenes,
- isoflavonoids,
- aminophenol derivatives of formula 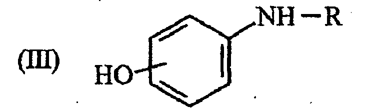
in which R is a radical corresponding to one of the formulae (i), (ii) or (iii) below
(i) -CO-NR₁R₂
(ii) -CO-O-R₃
(iii) -SO₂-R₃
in which
R₁ represents a hydrogen atom or a linear or branched, saturated or unsaturated, optionally hydroxylated C₁₋₆ alkyl radical,
R₂ represents a hydrogen atom or a radical chosen from saturated or unsaturated, linear, cyclic or branched, C₁₂ to C₃₀ optionally hydroxylated alkyl radicals, and
R₃ represents a radical chosen from saturated or unsaturated, linear, branched or cyclic C₁₂ to C₃₀ alkyl radicals, including fused polycyclic radicals, which are optionally hydroxylated.

13. Process according to Claim 12, **characterized in that** the salicylic acid derivatives are chosen from the 5-n-octanoylsalicylic, 5-n-decanoylsalicylic, 5-n-dodecanoylsalicylic, 5-n-octylsalicylic, 5-n-heptyloxysalicylic, 4-n-heptyloxysalicylic, 5-tert-octylsalicylic, 3-tert-butyl-5-methylsalicylic, 3-tert-butyl-6-methylsalicylic, 3,5-diisopropylsalicylic, 5-butoxysalicylic, 5-octyloxysalicylic, 5-propanoylsalicylic, 5-n-hexadecanoylsalicylic, 5-n-oleoylsalicylic and 5-benzoylsalicylic acid derivatives, monovalent and divalent salts thereof, and mixtures thereof.

14. Process according to Claim 12, **characterized in that** the sunscreens are chosen from anthranilates; cinnamic derivatives; dibenzoylmethane derivatives; salicylic derivatives; camphor derivatives; triazine derivatives, preferably 1,3,5-triazine derivatives; benzophenone derivatives; β,β'-diphenylacrylate derivatives; benzotriazole derivatives; benzalmalonate derivatives; benzimidazole derivatives; imidazolines; bis-benzazolyl derivatives; p-aminobenzoic acid (PABA) derivatives; methylenebis(hydroxyphenylbenzotriazole) derivatives; screening polymers and screening silicones; dimers derived from α-alkylstyrene; 4,4-diarylbutadienes, and mixtures thereof.

15. Process according to Claim 14 **characterized in that** the 1,3,5-triazine derivatives are chosen from the following compounds:
- 2-[(p-(tert-butylamido)anilino]-4,6-bis[(p-(2'-ethylhexyl-1'-oxycarbonyl) anilino]-1,3,5-triazine,
- 2,4,6-tris[p'-(2'-ethylhexyl-1'-oxycarbonyl)anilino]-1,3,5-triazine,
- 2,4-bis{[4-2-ethylhexyloxy]-2-hydroxy]phenyl}-6-(4-methoxyphenyl)-1,3,5-triazine,
- 2,4,6-tris(diisobutyl 4'-aminobenzalmalonate)-s-triazine, and mixtures thereof.

16. Process according to Claim 14, **characterized in that** the dibenzoylmethane derivative is butylmethoxydibenzoylmethane.

17. Process according to Claim 12, **characterized in that** the lipophilic compound is chosen from dehydroepiandrosterone (DHEA), DHEA sulphate, 7-hydroxy-DHEA, 7-keto-DHEA, prednisolone, prednisone, progesterone, pregnenolone, testosterone, diosgenin, hecogenin, ursolic acid, oleanolic acid, resveratrol and N-cholesteryloxycarbonyl-4-aminophenol, and isoflavonoids whose solubility in water at room temperature (25°C) is less than 0.01 %.

18. Cosmetic composition comprising at least one aqueous phase, at least one lipophilic compound dissolved in the aqueous phase, and an effective amount of at least one block amphiphilic copolymer comprising at least one ionic and/or at least one nonionic hydrophilic polymer block, and at least one hydrophobic polymer block obtained from at least one hydrophobic monomer chosen from :
- styrene and its derivatives such as 4-butylstyrene,
- vinyl acetate of formula CH₂=CH-OCOCH₃,
- vinyl ethers of formula CH₂=CHOR in which R is a linear or branched, saturated or unsaturated hydrocarbon-based radical containing from 1 to 6 carbon atoms,
- acrylonitrile,
- vinyl chloride and vinylidene chloride,
- caprolactam,
- alkenes such as ethylene, propylene, butylene and butadiene,
- alkylene oxides containing at least 4 carbon atoms and preferably from 4 to 6 carbon atoms,
- silicone derivatives producing, after polymerization, silicone polymers such as polydimethylsiloxane,
- hydrophobic vinyl monomers of formula (A) below: 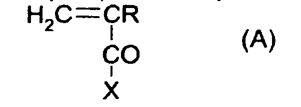
in which:
- R is chosen from H, -CH₃, -C₂H₅ and -C₃H₇,
- X is chosen from:
- alkyl oxides of -OR' type in which R' is a linear, cyclic or branched, saturated or unsaturated hydrocarbon-based radical containing from 1 to 22 carbon atoms, optionally substituted with a halogen atom (iodine, bromine, chlorine or fluorine); a sulphonic group (-SO₃⁻), a sulphate group (-SO₄⁻), a phosphate group (-PO₄H₂⁻); a hydroxyl group (-OH); a primary amine group (-NH₂); a secondary amine group (-NHR₁), a tertiary amine group (-NR₁R₂) or a quaternary amine group (-N⁺R₁R₂R₃) with R₁, R₂ and R₃ being, independently of each other, a linear or branched, saturated or unsaturated hydrocarbon-based radical containing from 1 to 22 carbon atoms, with the proviso that the sum of the carbon atoms of R' + R₁ + R₂ + R₃ does not exceed 22; R' may also be a perfluoroalkyl radical, preferably containing from 1 to 18 carbon atoms;
- -NH₂, -NHR' and -NR'R" groups in which R' and R" are, independently of each other, linear, cyclic or branched, saturated or unsaturated hydrocarbon-based radicals containing from 1 to 22 carbon atoms, with the proviso that the total number of carbon atoms of R' + R" does not exceed 22, R' and R" optionally being substituted with a halogen atom (iodine, bromine, chlorine or fluorine); a hydroxyl group (-OH); a sulphonic group (-SO₃⁻); a sulphate group (-SO₄⁻); a phosphate group (-PO₄H₂⁻); a primary amine group (-NH₂); a secondary amine group (-NHR₁), a tertiary amine group (-NR₁R₂) and/or a quaternary amine group (-N⁺R₁R₂R₃) with R₁, R₂ and R₃ being, independently of each other, a linear or branched, saturated or unsaturated hydrocarbon-based radical containing from 1 to 22 carbon atoms, with the proviso that the sum of the carbon atoms of R' + R" + R₁ + R₂ + R₃ does not exceed 22; R' and R" may also be perfluoroalkyl radicals, preferably containing from 1 to 18 carbon atoms.

19. Composition according to claim 18, **characterized in that** the weight concentration ratio between the lipophilic compound and the amphiphilic block copolymer is between 0.005 and 0.5 and preferably between 0.005 and 0.2.

20. Composition according to any one of Claims 18 to 19, **characterized in that** the lipophilic compound is present in the composition in weight contents of between 0.001 % and 10% of the total weight of the composition.

21. Composition according to any one of Claims 18 to 20, **characterized in that** it also contains one or more formulation adjuvants chosen from pigments, nanopigments, fatty substances, organic solvents, thickeners, softeners, free-radical scavengers, stabilizers, emollients, silicones, α-hydroxy acids, antifoams, moisturizers, vitamins, preserving agents, surfactants, fillers, sequestering agents, polymers, propellants, acidifying or basifying agents, dyes, opacifiers.

22. Composition according to any one of Claims 18 to 21, **characterized in that** it is a composition for protecting the human epidermis or the hair against ultraviolet rays, as antisun compositions; or is a makeup product; or is a composition for protecting the hair.

## Patentansprüche

1. Verfahren zum Lösen mindestens einer lipophilen Verbindung in einer wässrigen Phase, **dadurch gekennzeichnet, dass** es den Schritt umfasst, die lipophile Verbindung mit einer wirksamen Menge mindestens eines amphiphilen Blockcopolymers zu kombinieren, das mindestens einen ionischen und/oder mindestens einen nichtionischen hydrophilen Polymerblock und mindestens einen hydrophoben Polymerblock aufweist, der ausgehend von mindestens einem hydrophoben Monomer erhalten wird, das ausgewählt ist unter:
- Styrol und seinen Derivaten, wie 4-Butylstyrol,
- dem Vinylacetat der Formel CH₂=CH-OCOCH₃,
- Vinylethern der Formel CH₂=CHOR, wobei R eine lineare oder verzweigte, gesättigte oder ungesättigte Gruppe auf Kohlenwasserstoffbasis mit 1 bis 6 Kohlenstoffatomen ist,
- Acrylnitril,
- Vinylchlorid und Vinylidenchlorid,
- Caprolactam,
- Alkenen, wie Ethylen, Propylen, Butylen und Butadien,
- Alkylenoxiden mit mindestens 4 Kohlenstoffatomen und vorzugsweise 4 bis 6 Kohlenstoffatomen,
- Siliconderivaten, die nach der Polymerisation zu Siliconpolymeren wie Polydimethylsiloxan führen,
- hydrophoben Vinylmonomeren der folgenden Formel (A): 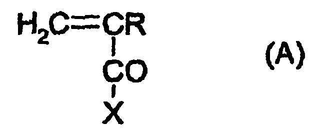
wobei in der Formel:
- R ausgewählt ist unter H, -CH₃, -C₂H₅ und -C₃H₇,
- X ausgewählt ist unter:
- Alkoxygruppen vom Typ -OR', wobei R' eine lineare, cyclische oder verzweigte, gesättigte oder ungesättigte Gruppe auf Kohlenwasserstoffbasis mit 1 bis 22 Kohlenstoffatomen ist, die gegebenenfalls mit einem Halogenatom (Jod, Brom, Chlor oder Fluor); einer Sulfonatgruppe (-SO₃-), einer Sulfatgruppe (-SO₄-), einer Phosphatgruppe (-PO₄H₂-); einer Hydroxygruppe (-OH); einer primären Aminogruppe (-NH₂); einer sekundären Aminogruppe (-NHR₁), einer tertiären Aminogruppe (-NR₁R₂) oder einer quartären Aminogruppe (-N⁺R₁R₂R₃) substituiert ist, wobei R₁, R₂ und R₃ unabhängig voneinander eine lineare oder verzweigte, gesättigte oder ungesättigte Gruppe auf Kohlenwasserstoffbasis mit 1 bis 22 Kohlenstoffatomen bedeuten, mit der Maßgabe, dass die Summe der Kohlenstoffatome von R' + R₁ + R₂ + R₃ 22 nicht übersteigt; R' kann auch eine Perfluoralkylgruppe bedeuten, die vorzugsweise 1 bis 18 Kohlenstoffatome aufweist;
- Gruppen -NH₂, -NHR' und -NR'R", wobei R' und R" unabhängig voneinander lineare, cyclische oder verzweigte, gesättigte oder ungesättigte Gruppen auf Kohlenwasserstoffbasis mit 1 bis 22 Kohlenstoffatomen bedeuten, mit der Maßgabe, dass die Gesamtzahl der Kohlenstoffatome von R' + R" 22 nicht übersteigt, wobei R' und R" gegebenenfalls substituiert sein können mit einem Halogenatom (Jod, Brom, Chlor oder Fluor); einer Hydroxygruppe (-OH); einer Sulfonatgruppe (-SO₃-); einer Sulfatgruppe (-SO₄-); einer Phosphatgruppe (-PO₄H₂-); einer primären Aminogruppe (-NH₂); einer sekundären Aminogruppe (-NHR₁), einer tertiären Aminogruppe (-NR₁R₂) und/oder einer quartären Aminogruppe (-N⁺R₁R₂R₃), wobei R₁, R₂ und R₃ unabhängig voneinander eine geradkettige oder verzweigte, gesättigte oder ungesättigte Gruppe auf Kohlenwasserstoffbasis mit 1 bis 22 Kohlenstoffatomen bedeuten, mit der Maßgabe, dass die Summe der Kohlenstoffatome von R' + R" + R₁ + R₂ + R₃ 22 nicht übersteigt; R' und R" können auch Perfluoralkylgruppen bedeuten, die vorzugsweise 1 bis 18 Kohlenstoffatome aufweisen.

2. Verfahren nach Anspruch 1, **dadurch gekennzeichnet, dass** es sich bei dem ionischen hydrophilen Polymerblock um Polyethylenimin handelt oder er ausgehend von wasserlöslichen Monomeren oder deren Salzen gebildet wird, die ausgewählt sind unter:
- (Meth)acrylsäure,
- Acrylamido-2-methylpropansulfonsäure (AMPS),
- Styrolsulfonsäure,
- Vinylsulfonsäure und (Meth)allylsulfonsäure,
- Vinylphosphonsäure,
- Maleinsäureanhydrid,
- Itaconsäure,
- Dimethyldiallylammoniumchlorid,
- quaternisiertem Dimethylaminoethylmethacrylat (DMAEMA),
- (Meth)acrylamidopropyltrimethylammoniumchlorid (APTAC und MAPTAC),
- Methylvinylimidazoliumchlorid,
- hydrophilen Vinylmonomeren der folgenden Formel (A): 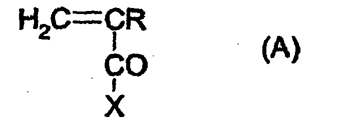
wobei in der Formel:
- R ausgewählt ist unter H, -CH₃, -C₂H₅ und -C₃H₇,
- X ausgewählt ist unter:
- Alkoxygruppen vom Typ -OR', wobei R' eine lineare, cyclische oder verzweigte, gesättigte oder ungesättigte Gruppe auf Kohlenwasserstoffbasis mit 1 bis 6 Kohlenstoffatomen bedeutet, die mit mindestens einer Sulfonatgruppe (-SO₃-) und/oder Sulfatgruppe (-SO₄-) und/oder Phosphatgruppe (-PO₄H₂-) und/oder quartären Aminogruppe (-N⁺R₁R₂R₃) substituiert ist, wobei R₁, R₂ und R₃ unabhängig voneinander eine lineare oder verzweigte, gesättigte oder ungesättigte Gruppe auf Kohlenwasserstoffbasis mit 1 bis 6 Kohlenstoffatomen bedeuten, mit der Maßgabe, dass die Summe von R' + R₁ + R₂ + R₃ 6 nicht übersteigt; wobei die Gruppe R' gegebenenfalls mit einem Halogenatom (Jod, Brom, Chlor oder Fluor); einer Hydroxygruppe (-OH); einer primären Aminogruppe (-NH₂); einer sekundären Aminogruppe (-NHR₁) oder einer tertiären Aminogruppe (-NR₁R₂) substituiert ist, wobei R₁ und R₂ unabhängig voneinander eine lineare oder verzweigte, gesättigte oder ungesättigte Gruppe auf Kohlenwasserstoffbasis mit 1 bis 6 Kohlenstoff atomen bedeuten, mit der Maßgabe, dass die Summe der Kohlenstoffatome von R' + R₁ + R₂ 6 nicht übersteigt,
- Gruppen -NH₂, -NHR' und -NR'R", wobei R' und R" unabhängig voneinander lineare, cyclische oder verzweigte, gesättigte oder ungesättigte Gruppen auf Kohlenwasserstoffbasis mit 1 bis 6 Kohlenstoffatomen bedeuten, mit der Maßgabe, dass die Gesamtzahl der Kohlenstoffatome von R' + R" 6 nicht übersteigt, wobei R' und/oder R" gegebenenfalls mit mindestens einer Sulfonatgruppe (-SO₃-) und/ oder Sulfatgruppe (-SO₄⁻) und/oder Phosphatgruppe (-PO₄H₂⁻) und/oder quartären Aminogruppe (-N⁺R₁R₂R₃) substituiert ist, wobei R₁, R₂ und R₃ unabhängig voneinander eine lineare oder verzweigte, gesättigte oder ungesättigte Gruppe auf Kohlenwasserstoffbasis mit 1 bis 6 Kohlenstoffatomen bedeuten, mit der Maßgabe, dass die Summe der Atome von R' + R₁ + R₂ + R₃ 6 nicht übersteigt; wobei die Gruppen R' und/oder R" gegebenenfalls mit einem Halogenatom (Jod, Brom, Chlor oder Fluor); einer Hydroxygruppe (-OH); einer primären Aminogruppe (-NH₂); einer sekundären Aminogruppe (-NHR₁) oder einer tertiären Aminogruppe (-NR₁R₂) substituiert sind, wobei R₁ und R₂ unabhängig voneinander eine lineare oder verzweigte, gesättigte oder ungesättigte Gruppe auf Kohlenwasserstoffbasis mit 1 bis 6 Kohlenstoffatomen bedeuten, mit der Maßgabe, dass die Summe der Kohlenstoffatome von R' + R" + R₁ + R₂ 6 nicht übersteigt.

3. Verfahren nach einem der vorhergehenden Ansprüche, **dadurch gekennzeichnet, dass** der ionische hydrophile Block unter (Meth)acrylsäure ausgewählt ist.

4. Verfahren nach einem der vorhergehenden Ansprüche, **dadurch gekennzeichnet, dass** der nichtionische hydrophile Polymerblock ein Block vom Polyoxyalkylentyp, beispielsweise vom Polyoxyethylentyp, oder Polyvinylpyrrolidon (PVP) ist oder ausgehend von wasserlöslichen Monomeren erhalten wird, die ausgewählt sind unter:
- (Meth)acrylamid,
- N-Vinylacetamid und N-Methyl-N-vinylacetamid,
- N-Vinylformamid und N-Methyl-N-vinylformamid,
- N-Vinyllactamen, die eine cyclische Alkylgruppe mit 4 bis 9 Kohlenstoffatomen enthalten, wie N-Vinylpyrrolidon, N-Butyrolactam und N-Vinylcaprolactam,
- dem Vinylalkohol der Formel CH₂=CHOH,
- Glycidyl(meth)acrylat,
- hydrophilen Vinylmonomeren der folgenden Formel (A): 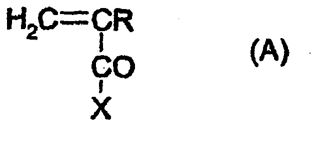
wobei in der Formel:
- R ausgewählt ist unter H, -CH3, -C2H5 und -C3H7,
- X ausgewählt ist unter:
- Alkoxygruppen vom Typ -OR', wobei R' eine lineare, cyclische oder verzweigte, gesättigte oder ungesättigte Gruppe auf Kohlenwasserstoffbasis mit 1 bis 6 Kohlenstoffatomen ist, die gegebenenfalls mit einem Halogenatom (Jod, Brom, Chlor oder Fluor); einer Hydroxygruppe (-OH); einer primären Aminogruppe (-NH₂); einer sekundären Aminogruppe (-NHR₁) oder einer tertiären Aminogruppe (-NR₁R₂) substituiert ist, wobei R₁ und R₂ unabhängig voneinander eine lineare oder verzweigte, gesättigte oder ungesättigte Gruppe auf Kohlenwasserstoffbasis mit 1 bis 6 Kohlenstoffatomen bedeuten, mit der Maßgabe, dass die Summe der Kohlenstoffatome von R' + R₁ + R₂ 6 nicht übersteigt,
- Gruppen -NH₂, -NHR' und -NR'R", wobei R' und R" unabhängig voneinander lineare, cyclische oder verzweigte, gesättigte oder ungesättigte Gruppen auf Kohlenwasserstoffbasis mit 1 bis 6 Kohlenstoffatomen bedeuten, mit der Maßgabe, dass die Gesamtzahl der Kohlenstoffatome von R' + R" 6 nicht übersteigt, wobei R' und R" gegebenenfalls mit einem Halogenatom (Jod, Brom, Chlor oder Fluor); einer Hydroxygruppe (-OH); einer primären Aminogruppe (-NH₂); einer sekundären Aminogruppe (-NHR₁) oder einer tertiären Aminogruppe (-NR₁R₂) substituiert sind, wobei R₁ und R₂ unabhängig voneinander eine lineare oder verzweigte, gesättigte oder ungesättigte Gruppe auf Kohlenwasserstoffbasis mit 1 bis 6 Kohlenstoffatomen bedeuten, mit der Maßgabe, dass die Summe der Kohlenstoffatome von R' + R" + R₁ + R₂ 6 nicht übersteigt.

5. Verfahren nach Anspruch 4, **dadurch gekennzeichnet, dass** der nichtionische hydrophile Polymerblock unter Polyethylenoxid und Polyvinylpyrrolidon ausgewählt ist.

6. Verfahren nach einem der vorhergehenden Ansprüche, **dadurch gekennzeichnet, dass** der hydrophile Polymerblock nichtionisch ist.

7. Verfahren nach einem der vorhergehenden Ansprüche, **dadurch gekennzeichnet, dass** der hydrophobe Polymerblock ausgehend von mindestens einem hydrophoben Monomer gebildet wird, das ausgewählt ist unter Styrol, *tert*-Butylstyrol, Methylmethacrylat, Ethyl(meth)acrylat), *n*-Butyl(meth)acrylat, *tert*-Butyl(meth)acrylat, Cyclohexylacrylat, Isobornylacrylat, 2-Ethylhexylacrylat, Ethylperfluoroctylacrylat, Trifluormethyl(meth)acrylat, Polybutylenoxid oder Polyoxybutylen (POB), Butadien, Ethylen, Propylen und Butylen.

8. Verfahren nach einem der vorhergehenden Ansprüche, **dadurch gekennzeichnet, dass** das Blockcopolymer unter den folgenden Blockcopolymeren ausgewählt ist:
- Polystyrol/Polyoxyethylen
- Polymethylmethacrylat/Polyoxyethylen
- Polybutylmethacrylat/Polyoxyethylen
- Polyoxybutylen/Polyoxyethylen
- Polycaprolacton/Polyoxyethylen
- Polyethylen/Polyoxyethylen
- Polyoxyethylen/Polyoxybutylen/Polyoxyethylen.

9. Verfahren nach einem der vorhergehenden Ansprüche, **dadurch gekennzeichnet, dass** das Molekulargewicht des Blockcopolymers im Bereich von 1 000 bis 500 000 und vorzugsweise 2 000 bis 100 000 liegt.

10. Verfahren nach einem der vorhergehenden Ansprüche, **dadurch gekennzeichnet, dass** das Gewichtsverhältnis A/B des hydrophilen Blocks A und des hydrophoben Blocks B im Bereich von 1/100 bis 50/1 liegt.

11. Verfahren nach einem der vorhergehenden Ansprüche, **dadurch gekennzeichnet, dass** das Gewichtsverhältnis der lipophilen Verbindung und des amphiphilen Blockcopolymers im Bereich von 0,005 bis 0,5 und vorzugsweise 0,005 bis 0,2 liegt.

12. Verfahren nach einem der vorhergehenden Ansprüche, **dadurch gekennzeichnet, dass** die lipophile Verbindung ausgewählt ist unter:
- Emollientien, entzündungshemmenden Wirkstoffen, antibakteriellen Wirkstoffen, antimykotischen Wirkstoffen, antiviralen Wirkstoffen, antiseborrhoischen Wirkstoffen, Wirkstoffen gegen Akne, Keratolytika, Antihistaminika, Anästhetika, Wundheilungsmitteln, Wirkstoffen, die die Pigmentierung modifizieren, Bräunungsbeschleunigern, Stoffen für die künstliche Bräunung, Liporegulatoren, Wirkstoffen gegen Alterung und Wirkstoffen gegen Falten, erfrischenden Stoffen, vaskularen Schutzmitteln, Insekten abwehrenden Wirkstoffen, Deodorants, Antischuppenmitteln, Wirkstoffen gegen Haarausfall,
- etherischen Ölen, die insbesondere unter Eukalyptusöl, Lavandinöl, Lavendelöl, Vetiveröl, Öl aus Litsea cubeba, Citronenöl, Sandelholzöl, Rosmarinöl, Kamillenöl, Pfefferkrautöl, Muskatnussöl, Zimtöl, Ysopöl, Kümmelöl, Orangenöl, Geraniolöl und Kadeöl, Duftstoffen;
- Sonnenschutzfiltern, Antioxidantien, Radikalfängern für freie Radikale und Feuchthaltemitteln;
- Vitaminen wie Vitamin A (Retinol) und seinen Estern, Vitamin E oder seinen Estern, wie Tocopherylacetat, Vitamin D oder seinen Derivaten und Vitamin F oder seinen Derivaten; Carotinen wie β-Carotin und deren Derivaten, wie Lycopin, und Salicylsäurederivaten;
- Dehydroepiandrosteron (DHEA) und seinen biologischen Precursoren und deren Derivaten, wobei Cholesterin und seine Ester und Pflanzensterole, wie Phytosterole und Sitosterole, und deren Ester ausgenommen sind,
- pentacyclische Triterpensäuren, wie Ursolsäure und Oleanolsäure,
- Hydroxystilbenen,
- Isoflavonoiden,
- Aminophenolderivaten der Formel 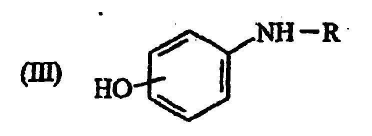
worin R eine Gruppe der folgenden Formeln (i), (ii) oder (iii) bedeutet:
(i) -CO-NR₁R₂
(ii) -CO-O-R₃
(iii) -SO₂-R₃
wobei in den Formeln bedeuten:
R₁ ein Wasserstoffatom oder eine geradkettige oder verzweigte, gesättigte oder ungesättigte, gegebenenfalls hydroxylierte C₁₋₆-Alkylgruppe,
R₂ ein Wasserstoffatom oder eine Gruppe, die unter den gesättigten oder ungesättigten, linearen, cyclischen oder verzweigten, gegebenenfalls hydroxylierten C₁₂₋₃₀-Allylgruppen ausgewählt ist, und
R₃ eine Gruppe, die unter den gesättigten oder ungesättigten, linearen, verzweigten oder cyclischen C₁₂₋₃₀-Alkylgruppen, einschließlich kondensierten polycyclischen Gruppen, die gegebenenfalls hydroxyliert sind, ausgewählt ist.

13. Verfahren nach Anspruch 12, **dadurch gekennzeichnet, dass** die Salicylsäurederivate ausgewählt sind unter 5-*n*-Octanoylsalicylsäurederivaten, 5-*n*-Decanoylsalicylsäurederivaten, 5-*n*-Dodecanoylsalicylsäurederivaten, 5-*n*-Octylsalicylsäurederivaten, 5-*n*-Heptyloxysalicylsäurederivaten, 4-*n*-Heptyloxysalicylsäurederivaten, 5-*tert*-Octylsalicylsäurederivaten, 3-*tert*-Butyl-5-methylsalicylsäurederivaten, 3-*tert*-Butyl-6-methylsalicylsäurederivaten, 3,5-Diisopropylsalicylsäurederivaten, 5-Butoxysalicylsäurederivaten, 5-Octyloxysalicylsäurederivaten, 5-Propanoylsalicylsäurederivaten, 5-*n*-Hexadecanoylsalicylsäurederivaten, 5-*n-*Oleoylsalicylsäurederivaten und 5-Benzoylsalicylsäurederivaten, einwertigen und zweiwertigen Salzen dieser Verbindungen und deren Gemischen.

14. Verfahren nach Anspruch 12, **dadurch gekennzeichnet, dass** die Sonnenschutzfilter unter den Anthranilaten; Zimtsäurederivaten; Dibenzoylmethanderivaten; Salicylsäurederivaten; Campherderivaten; Triazinderivaten, vorzugsweise 1,3,5-Triazinderivaten; Benzophenonderivaten; β,β'-Diphenylacrylatderivaten; Benzotriazolderivaten; Benzalmalonatderivaten; Benzimidazolderivaten; Imidazolinen; Bis-benzazolylderivaten; Derivaten von p-Aminobenzoesäure (PABA); Methylenbis(hydroxyphenylbenzotriazol)derivaten; Filterpolymeren und Filtersiliconen; von α-Alkylstyrol abgeleiteten Dimeren; 4,4-Diarylbutadienen und deren Gemischen ausgewählt sind.

15. Verfahren nach Anspruch 14, **dadurch gekennzeichnet, dass** die 1,3,5-Triazinderivate unter den folgenden Verbindungen ausgewählt sind:
- 2-[(*p*-*tert*-Butylamido)anilino]-4,6-bis[(*p*-(2'-ethylhexyl-1'-oxycarbonyl)anilino]-1,3,5-triazin,
- 2,4,6-Tris[p'-(2'-ethylhexyl-1'-oxycarbonyl)anilino]-1,3,5-triazin,
- 2,4-Bis{[4-2-ethylhexyloxy]-2-hydroxy]phenyl}-6-(4-methoxyphenyl)-1,3, 5-triazin,
- 2,4,6-Tris(diisobutyl-4'-aminobenzalmalonat)-s-triazin, und deren Gemischen.

16. Verfahren nach Anspruch 14, **dadurch gekennzeichnet, dass** das Dibenzoylmethanderivat das Butylmethoxydibenzoylmethan ist.

17. Verfahren nach Anspruch 12, **dadurch gekennzeichnet, dass** die lipophile Verbindung unter Dehydroepiandrosteron (DHEA), DHEA-Sulfat, 7-Hydroxy-DHEA, 7-Keto-DHEA, Prednisolon, Prednison, Progesteron, Pregnenolon, Testosteron, Diosgenin, Hecogenin, Ursolsäure, Oleanolsäure, Resveratrol und N-Cholesteryloxycarbonyl-4-aminophenol und Isoflavonoiden ausgewählt ist, deren Wasserlöslichkeit bei Raumtemperatur (25 °C) weniger als 0,01 % ist.

18. Kosmetische Zusammensetzung, die mindestens eine wässrige' Phase, mindestens eine in der wässrigen Phase gelöste lipophile Verbindung und eine wirksame Menge mindestens eines amphiphilen Blockcopolymers enthält, das mindestens einen ionischen und/ oder mindestens einen nichtionischen hydrophilen Polymerblock und mindestens einen hydrophoben Polymerblock aufweist, der ausgehend von mindestens einem hydrophoben Monomer erhalten wird, das ausgewählt ist unter:
- Styrol und seinen Derivaten, wie 4-Butylstyrol,
- dem Vinylacetat der Formel CH₂=CH-OCOCH₃,
- Vinylethern der Formel CH₂=CHOR, wobei R eine geradkettige oder verzweigte, gesättigte oder ungesättigte Gruppe auf Kohlenwasserstoffbasis mit 1 bis 6 Kohlenstoffatomen ist,
- Acrylnitril,
- Vinylchlorid und Vinylidenchlorid,
- Caprolactam,
- Alkenen, wie Ethylen, Propylen, Butylen und Butadien,
- Alkylenoxiden mit mindestens 4 Kohlenstoffatomen und vorzugsweise 4 bis 6 Kohlenstoffatomen,
- Siliconderivaten, die nach der Polymerisation zu Siliconpolymeren führen, wie Polydimethylsiloxan,
- hydrophoben Vinylmonomeren der folgenden Formel (A): 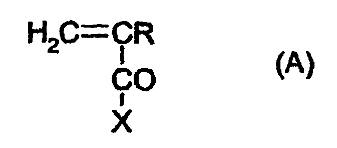
wobei in der Formel:
- R ausgewählt ist unter H, -CH3, -C2H5 und -C3H7,
- X ausgewählt ist unter:
- Alkoxygruppen vom Typ -OR', wobei R' eine lineare, cyclische oder verzweigte, gesättigte oder ungesättigte Gruppe auf Kohlenwasserstoffbasis mit 1 bis 22 Kohlenstoffatomen ist, die gegebenenfalls mit einem Halogenatom (Jod, Brom, Chlor oder Fluor); einer Sulfonatgruppe (-SO₃-), einer Sulfatgruppe (-SO₄-), einer Phosphatgruppe (-PO₄H₂-); einer Hydroxygruppe (-OH); einer primären Aminogruppe (-NH₂); einer sekundären Aminogruppe (-NHR₁), einer tertiären Aminogruppe (-NR₁R₂) oder einer quartären Aminogruppe (-N⁺R₁R₂R₃) substituiert ist, wobei R₁, R₂ und R₃ unabhängig voneinander eine lineare oder verzweigte, gesättigte oder ungesättigte Gruppe auf Kohlenwasserstoffbasis mit 1 bis 22 Kohlenstoffatomen bedeuten, mit der Maßgabe, dass die Summe der Kohlenstoffatome von R' + R₁ + R₂ + R₃ 22 nicht übersteigt; R' kann auch eine Perfluoralkylgruppe bedeuten, die vorzugsweise 1 bis 18 Kohlenstoffatome aufweist;
- Gruppen -NH₂, -NHR' und -NR'R", wobei R' und R" unabhängig voneinander lineare, cyclische oder verzweigte, gesättigte oder ungesättigte Gruppen auf Kohlenwasserstoffbasis mit 1 bis 22 Kohlenstoffatomen bedeuten, mit der Maßgabe, dass die Gesamtzahl der Kohlenstoffatome von R' + R" 22 nicht übersteigt, wobei R' und R" gegebenenfalls substituiert sein können mit einem Halogenatom (Jod, Brom, Chlor oder Fluor); einer Hydroxygruppe (-OH); einer Sulfonatgruppe (-SO₃-); einer Sulfatgruppe (-SO₄-); einer Phosphatgruppe (-PO₄H₂-); einer primären Aminogruppe (-NH₂); einer sekundären Aminogruppe (-NHR₁), einer tertiären Aminogruppe (-NR₁R₂) und/oder einer quartären Aminogruppe (-N⁺R₁R₂R₃), wobei R₁, R₂ und R₃ unabhängig voneinander eine geradkettige oder verzweigte, gesättigte oder ungesättigte Gruppe auf Kohlenwasserstoffbasis mit 1 bis 22 Kohlenstoffatomen bedeuten, mit der Maßgabe, dass die Summe der Kohlenstoffatome von R' + R" + R₁ + R₂ + R₃ 22 nicht übersteigt; R' und R" können auch Perfluoralkylgruppen bedeuten, die vorzugsweise 1 bis 18 Kohlenstoffatome aufweisen.

19. Zusammensetzung nach Anspruch 18, **dadurch gekennzeichnet, dass** das Gewichtsverhältnis der lipophilen Verbindung und des amphiphilen Blockcopolymers im Bereich von 0,005 bis 0,5 und vorzugsweise 0,005 bis 0,2 liegt.

20. Zusammensetzung nach einem der Ansprüche 18 bis 19, **dadurch gekennzeichnet, dass** die lipophile Verbindung in der Zusammensetzung in Mengenanteilen von 0,001 bis 10 Gew.-% des Gesamtgewichts der Zusammensetzung enthalten ist.

21. Zusammensetzung nach einem der Ansprüche 18 bis 20, **dadurch gekennzeichnet, dass** sie ferner einen oder mehrere Hilfsstoffe für die Formulierung enthält, die ausgewählt sind unter Pigmenten, Nanopigmenten, Fettsubstanzen, organischen Lösungsmitteln, Verdickungsmitteln, Weichmachern, Radikalfängern für freie Radikale, Stabilisatoren, Emollientien, Siliconen, α-Hydroxysäuren, Schaumverhütungsmitteln, Feuchthaltemitteln, Vitaminen, Konservierungsmitteln, grenzflächenaktiven Stoffen, Füllstoffen, Maskierungsmitteln, Polymeren, Treibmitteln, Ansäuerungsmitteln oder Alkalisierungsmitteln, Farbmitteln, Trübungsmitteln.

22. Zusammensetzung nach einem der Ansprüche 18 bis 21, **dadurch gekennzeichnet, dass** es sich um eine Zusammensetzung zum Schutz der menschlichen Epidermis oder der Haare gegen UV-Strahlung, wie Sonnenschutzzusammensetzungen; oder ein Make-up-Produkt; oder eine Zusammensetzung zum Schutz der Haare handelt.

## Revendications

1. Procédé de solubilisation d'au moins un composé lipophile dans une phase aqueuse, **caractérisé en ce qu'**il consiste à associer audit composé lipophile une quantité efficace d'au moins un copolymère bloc amphiphile comportant au moins un bloc polymérique hydrophile ionique et/ou non ionique, et au moins un bloc polymérique hydrophobe obtenu à partir d'au moins un monomère hydrophobe choisi parmi :
- le styrène et ses dérivés tels que le 4-butylstyrène,
- l'acétate de vinyle de formule CH₂=CH-OCOCH₃,
- les vinyléthers de formule CH₂=CHOR dans laquelle R est un radical hydrocarboné, linéaire ou ramifié, saturé ou insaturé, ayant de 1 à 6 atomes de carbone,
- l'acrylonitrile,
- le chlorure de vinyle et le chlorure de vinylidène,
- le caprolactame,
- les alcènes tels que l'éthylène, le propylène, le butylène et le butadiène,
- les oxydes d'alkylène comportant au moins 4 atomes de carbone, et de préférence de 4 à 6 atomes de carbone,
- les dérivés siliconés, conduisant après polymérisation à des polymères siliconés tels que le polydiméthylsiloxane,
- les monomères vinyliques hydrophobes de formule (A) suivante : 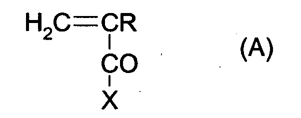
dans laquelle :
- R est choisi parmi H, -CH₃, -C₂H₅ ou -C₃H₇,
- X est choisi parmi :
- les oxydes d'alkyle de type -OR' où R' est un radical hydrocarboné, linéaire, cyclique ou ramifié, saturé ou insaturé, ayant de 1 à 22 atomes de carbone, éventuellement substitué par un atome d'halogène (iode, brome, chlore ou fluor); un groupement sulfonique (-SO₃⁻), sulfate (-SO₄⁻), phosphate (-PO₄H₂⁻); hydroxyle (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant de 1 à 22 atomes de carbone, sous réserve que la somme des atomes de carbone de R' + R₁ + R₂ + R₃ ne dépasse pas 22; R' pouvant également être un radical perfluoroalkyle, de préférence ayant de 1 à 18 atomes de carbone;
- les groupements -NH₂, -NHR' et -NR'R" dans lesquels R' et R" sont, indépendamment l'un de l'autre, des radicaux hydrocarbonés, linéaires, cycliques ou ramifiés, saturés ou insaturés, ayant de 1 à 22 atomes de carbone, sous réserve que le nombre total d'atomes de carbone de R' + R" ne dépasse pas 22, R' et R" étant éventuellement substitués par un atome d'halogène (iode, brome, chlore ou fluor) ; un groupement hydroxyle (-OH) ;sulfonique (-SO₃⁻) ; sulfate (-SO₄⁻); phosphate (-PO₄H₂⁻) ; amine primaire (-NH₂) ; amine secondaire (-NHR₁), tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant de 1 à 22 atomes de carbone, sous réserve que la somme des atomes de carbone de R' + R" + R₁ + R₂ + R₃ ne dépasse pas 22; R' et R" pouvant également être des radicaux perfluoroalkyles, de préférence ayant de 1 à 18 atomes de carbone.

2. Procédé selon la revendication 1, **caractérisé en ce que** le bloc polymérique hydrophile ionique est la polyéthylèneimine ou est obtenu à partir de monomères hydrosolubles ou de leurs sels, choisis parmi :
- l'acide (méth)acrylique,
- l'acide acrylamido-2-méthylpropane sulfonique (AMPS),
- l'acide styrènesulfonique,
- l'acide vinylsulfonique et l'acide (méth)allylsulfonique,
- l'acide vinylphophonique,
- l'anhydride maléique,
- l'acide itaconique,
- le chlorure de diméthyldiallyl ammonium,
- le méthacrylate de diméthylaminoéthyle quaternisé (MADAME),
- le chlorure de (méth)acrylamidopropyltriméthyle ammonium (APTAC et MAPTAC),
- le chlorure de méthylvinylimidazolium,
- les monomères vinyliques hydrophiles de formule (A) suivante : 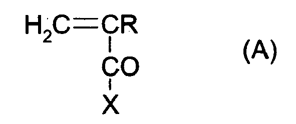
dans laquelle :
- R est choisi parmi H, -CH₃, -C₂H₅ ou -C₃H₇,
- X est choisi parmi :
- les oxydes d'alkyle de type -OR' où R' est un radical hydrocarboné, linéaire, cyclique ou ramifié, saturé ou insaturé, ayant de 1 à 6 atomes de carbone, substitué par au moins un groupement sulfonique (-SO₃⁻) et/ou sulfate (-SO₄⁻) et/ou phosphate (-PO₄H₂⁻) et/ou amine quaternaire (-N⁺R₁R₂R₃) avec R₁, R₂, R₃ étant, indépendamment l'un de l'autre, un radical hydrocarboné, linéaire ou ramifié, saturé ou insaturé, ayant de 1 à 6 atomes de carbone, sous réserve que la somme des atomes de R' + R₁ + R₂ +R₃ ne dépasse pas 6; le radical R' est éventuellement substitué par un atome d'halogène (iode, brome, chlore ou fluor) ; un groupement hydroxyle (-OH); amine primaire (-NH₂); amine secondaire (-NHR₁), amine tertiaire (-NR₁R₂) avec R₁, R₂ étant, indépendamment l'un de l'autre, un radical hydrocarboné, linéaire ou ramifié, saturé ou insaturé ayant de 1 à 6 atomes de carbone, sous réserve que la somme des atomes de carbone de R' + R₁ + R₂ ne dépasse pas 6;
- les groupements -NH₂, -NHR' et -NR'R" dans lesquels R' et R" sont, indépendamment l'un de l'autre, des radicaux hydrocarbonés, linéaires, cycliques ou ramifiés, saturés ou insaturés, ayant de 1 à 6 atomes de carbone, sous réserve que le nombre total d'atomes de carbone de R' + R" ne dépasse pas 6, R' et/ou R" étant éventuellement substitués par au moins un groupement sulfonique (-SO₃⁻) et/ou sulfate (-SO₄⁻) et/ou phosphate (-PO₄H₂⁻) et/ou amine quaternaire (-N⁺R₁R₂R₃) avec R₁, R₂, R₃ étant, indépendamment l'un de l'autre, un radical hydrocarboné, linéaire ou ramifié, saturé ou insaturé, ayant de 1 à 6 atomes de carbone, sous réserve que la somme des atomes de R' + R₁ + R₂ +R₃ ne dépasse pas 6; les radicaux R' et/ou R" étant éventuellement substitués par un atome d'halogène (iode, brome, chlore ou fluor) ; un groupement hydroxyle (-OH) ; amine primaire (-NH₂) ; amine secondaire (-NHR₁), amine tertiaire (-NR₁R₂) avec R₁, R₂ étant, indépendamment l'un de l'autre, un radical hydrocarboné, linéaire ou ramifié, saturé ou insaturé ayant de 1 à 6 atomes de carbone, sous réserve que la somme des atomes de carbone de R' + R" + R₁ + R₂ ne dépasse pas 6.

3. Procédé selon l'une quelconque des revendications précédentes, **caractérisé en ce que** le bloc hydrophile ionique est choisi parmi l'acide (méth)acrylique.

4. Procédé selon l'une quelconque des revendications précédentes, **caractérisé en ce que** le bloc polymérique hydrophile non ionique est du type polyoxyalkylené, par exemple polyoxyéthylène, ou est polyvinylpyrrolidone (PVP), ou est obtenu à partir de monomères hydrosolubles choisis parmi :
- le (méth)acrylamide,
- la N-vinylacétamide et la N-méthyl N-vinylacétamide,
- la N-vinylformamide et la N-méthyl N-vinylformamide,
- les N-vinyllactames comportant un groupe alkyle cyclique ayant de 4 à 9 atomes de carbone, tels que la N-vinylpyrrolidone, la N-butyrolactame et la N-vinylcaprolactame,
- l'alcool vinylique de formule CH₂=CHOH,
- le (méth)acrylate de glycidyle,
- les monomères vinyliques hydrophiles de formule (A) suivante : 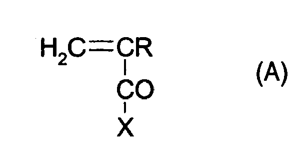
dans laquelle :
- R est choisi parmi H, -CH₃, -C₂H₅ ou -C₃H₇,
- X est choisi parmi:
- les oxydes d'alkyle de type -OR' où R' est un radical hydrocarboné, linéaire, cyclique ou ramifié, saturé ou insaturé, ayant de 1 à 6 atomes de carbone, éventuellement substitué par un atome d'halogène (iode, brome, chlore ou fluor) ; un groupement hydroxyle (-OH) ; amine primaire (-NH₂) ; amine secondaire (-NHR₁), ou amine tertiaire (-NR₁R₂) avec R₁, R₂ étant, indépendamment l'un de l'autre, un radical hydrocarboné, linéaire ou ramifié, saturé ou insaturé ayant de 1 à 6 atomes de carbone, sous réserve que la somme des atomes de carbone de R' + R₁ + R₂ ne dépasse pas 6 ;
- les groupements -NH₂, -NHR' et -NR'R" dans lesquels R' et R" sont, indépendamment l'un de l'autre des radicaux hydrocarbonés, linéaires, cycliques ou ramifiés, saturés ou insaturés, ayant de 1 à 6 atomes de carbone, sous réserve que le nombre total d'atomes de carbone de R' + R" ne dépasse pas 6, R' et R" étant éventuellement substitués par un atome d'halogène (iode, brome, chlore ou fluor) ; un groupement hydroxyle (-OH) ; amine primaire (-NH₂) ; amine secondaire (-NHR₁), ou amine tertiaire (-NR₁R₂) avec R₁ et R₂ étant, indépendamment l'un de l'autre, un radical hydrocarboné, linéaire ou ramifié, saturé ou insaturé ayant de 1 à 6 atomes de carbone, sous réserve que la somme des atomes de carbone de R' + R" + R₁ + R₂ ne dépasse pas 6.

5. Procédé selon la revendication 4, **caractérisé en ce que** le bloc polymérique hydrophile non ionique est choisi parmi le polyoxyde d'éthylène et la polyvinylpyrrolidone.

6. Procédé selon l'une quelconque des revendications précédentes, **caractérisé en ce que** le bloc polymérique hydrophile est non ionique.

7. Procédé selon l'une quelconque des revendications précédentes, **caractérisé en ce que** le bloc polymérique hydrophobe est obtenu à partir d'au moins un monomère hydrophobe choisi parmi le styrène, le tert-butylstyrène, le méthacrylate de méthyle, le (méth)acrylate d'éthyle, le (méth)acrylate de n-butyle; le (méth)acrylate de tert-butyle; l'acrylate de cyclohexyle; l'acrylate d'isobornyle; l'acrylate d'éthyl-2-hexyle; l'acrylate d'éthyl perfluorooctyle; le (méth)acrylate de trifluorométhyle; le polybutylène oxyde ou polyoxybutylène (POB); le butadiène, l'éthylène, le propylène et le butylène.

8. Procédé selon l'une quelconque des revendications précédentes, **caractérisé en ce que** le copolymère bloc est choisi parmi les copolymères blocs suivants :
- polystyrène/polyoxyéthylène
- polyméthylméthacrylate/polyoxyéthylène
- polybutylméthacrylate/polyoxyéthylène
- polyoxybutylène/polyoxyéthylène
- polycaprolactone/polyoxyéthylène
- polyéthylène/polyoxyéthylène
- polyoxyéthylène/polyoxybutylène/polyoxyéthylène.

9. Procédé selon l'une quelconque des revendications précédentes, **caractérisé en ce que** le poids moléculaire du copolymère bloc est compris entre 1000 et 500 000, de préférence entre 2000 et 100 000.

10. Procédé selon l'une quelconque des revendications précédentes, **caractérisé en ce que** le rapport en poids A/B entre le bloc hydrophile A et le bloc hydrophobe B est compris entre 1/100 et 50/1.

11. Procédé selon l'une quelconque des revendications précédentes, **caractérisé en ce que** le rapport de concentration en poids entre le composé lipophile et le copolymère bloc amphiphile est compris entre 0,005 et 0,5, de préférence entre 0,005 et 0,2.

12. Procédé selon l'une quelconque des revendications précédentes, **caractérisé en ce que** le composé lipophile est choisi parmi :
- les agents émollients, les anti-inflammatoires, les anti-bactériens, les antifongiques, les anti-viraux, les anti-séborrhéiques, les anti-acnéiques, les kératolytiques, les anti-histaminiques, les anesthésiques, les agents cicatrisants, les modificateurs de la pigmentation, les accélérateurs de bronzage, les agents de brunissage artificiel, les liporégulateurs, les agents anti-vieillissement et antirides, les agents rafraîchissants, les protecteurs vasculaires, les répulsifs d'insectes, les déodorants, les agents anti-pelliculaires, les agents anti-chute de cheveux,
- les huiles essentielles, pouvant notamment être choisies parmi les huiles d'eucalyptus, de lavandin, de lavande, de vétiver, de Litsea cubeba, de citron, de santal, de romarin, de camomille, de sarriette, de noix de muscade, de cannelle, d'hysope, de carvi, d'orange, de géraniol, et de cade, les parfums,
- les filtres solaires, les anti-oxydants, les piégeurs de radicaux libres, les agents hydratants;
- les vitamines telles que la vitamine A (rétinol) ou des esters de celle-ci, la vitamine E ou des esters de celle-ci tels que l'acétate de tocophérol, la vitamine D ou des dérivés de celle-ci et la vitamine F ou de dérivés de celle-ci; les carotènes tels que le β-carotène et les dérivés de ceux-ci tels le lycopène et les dérivés de l'acide salicylique;
- la déhydroépiandrostérone (DHEA) et ses précurseurs et dérivés biologiques, à l'exception du cholestérol et de ses esters et des stérols végétaux tels que les phytostérols et sitostérols, et de leurs esters,
- les acides triterpène pentacycliques tels que l'acide ursolique et l'acide oléanolique,
- les hydroxystilbènes,
- les isoflavonoïdes,
- les dérivés d'aminophénol de formule : 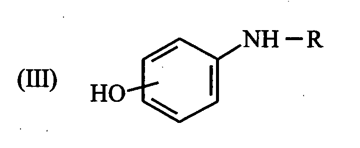
dans laquelle R est un radical correspondant à l'une des formules (i), (ii) ou (iii) suivantes
(i) -CO-NR₁R₂
(ii) -CO-O-R₃
(iii) - SO₂-R₃
où
R₁ représente un atome d'hydrogène ou un radical alkyle en C₁₋₆, linéaire ou ramifié, saturé ou insaturé, éventuellement hydroxylé,
R₂ représente un atome d'hydrogène ou un radical choisi parmi les radicaux alkyle, saturés ou insaturés, linéaires, cycliques ou ramifiés, en C₁₂ à C₃₀, éventuellement hydroxylés, et
R₃ représente un radical choisi parmi les radicaux alkyle en C₁₂ à C₃₀, saturés ou insaturés, linéaires, ramifiés ou cycliques, y compris polycycliques condensés, et éventuellement hydroxylés.

13. Procédé selon la revendication 12, **caractérisé en ce que** les dérivés de l'acide salicylique sont choisis parmi les dérivés d'acide n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents et leurs mélanges.

14. Procédé selon la revendication 12, **caractérisé en ce que** les filtres solaires sont choisis parmi les anthranilates ; les dérivés cinnamiques ; les dérivés de dibenzoylméthane ; les dérivés salicyliques; les dérivés du camphre ; les dérivés de triazine, de préférence les dérivés de 1,3,5-triazine ; les dérivés de la benzophénone ; les dérivés de β-β'-diphénylacrylate ; les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole); les polymères filtres et silicones filtres ; les dimères dérivés d'α-alkylstyrène ; les 4,4-diarylbutadiènes et leurs mélanges.

15. Procédé selon la revendication 14, **caractérisé en ce que** les dérivés de 1,3,5-triazine sont choisis parmi les composés suivants :
- la 2-[(p-(tértiobutylamido)anilino]-4,6-bis-[(p-(2'-éthylhexyl-1'-oxycarbonyl) anilino]-1,3,5-triazine,
- la 2,4,6-tris[p'-(2'-éthylhexyl-1'-oxycarbonyl)anilino]-1,3,5-triazine,
- la 2,4-bis{[4-2-éthyl-hexyloxy]-2-hydroxy]-phenyl}-6-(4-méthoxy-phenyl)-1,3,5-triazine,
- la 2,4,6-tris-(4'-amino benzalmalonate de diisobutyle)-s-triazine, et leurs mélanges.

16. Procédé selon la revendication 14, **caractérisé en ce que** le dérivé de dibenzoylméthane est le butyl méthoxydibenzoylméthane.

17. Procédé selon la revendication 12, **caractérisé en ce que** le composé lipophile est choisi parmi la déhydroépiandrostérone (DHEA), le sulfate de DHEA, la 7-hydroxy-DHEA, la 7-céto-DHEA, la prednisolone, la prednisone, la progestérone, la prégnénolone, la testostérone, la diosgénine, l'hécogénine, l'acide ursolique, l'acide oléanolique, le resvératrol et le N-cholestéryloxycarbonyl-4-aminophénol, et les isoflavonoïdes dont la solubilité dans l'eau à température ambiante (25°C) est inférieure à 0,01%.

18. Composition cosmétique comprenant au moins une phase aqueuse, au moins un composé lipophile dissous dans la phase aqueuse et une quantité efficace d'au moins un copolymère bloc amphiphile comportant au moins un bloc polymérique hydrophile ionique et/ou non ionique, et au moins un bloc polymérique hydrophobe obtenu à partir d'au moins un monomère hydrophobe choisi parmi :
- le styrène et ses dérivés tels que le 4-butylstyrène,
- l'acétate de vinyle de formule CH₂=CH-OCOCH₃,
- les vinyléthers de formule CH₂=CHOR dans laquelle R est un radical hydrocarboné, linéaire ou ramifié, saturé ou insaturé, ayant de 1 à 6 atomes de carbone,
- l'acrylonitrile,
- le chlorure de vinyle et le chlorure de vinylidène,
- le caprolactame,
- les alcènes tels que l'éthylène, le propylène, le butylène et le butadiène,
- les oxydes d'alkylène comportant plus de 4 atomes de carbone, et de préférence de 4 à 6 atomes de carbone,
- les dérivés siliconés, conduisant après polymérisation à des polymères siliconés tels que le polydiméthylsiloxane,
- les monomères vinyliques hydrophobes de formule (A) suivante : 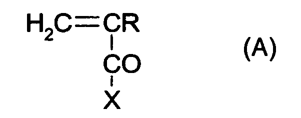
dans laquelle :
- R est choisi parmi H, -CH₃, -C₂H₅ ou -C₃H₇,
- X est choisi parmi :
- les oxydes d'alkyle de type -OR' où R' est un radical hydrocarboné, linéaire, cyclique ou ramifié, saturé ou insaturé, ayant de 1 à 22 atomes de carbone, éventuellement substitué par un atome d'halogène (iode, brome, chlore ou fluor) ; un groupement sulfonique (-SO₃⁻), sulfate (-SO₄⁻), phosphate (-PO₄H₂⁻) ; hydroxyle (-OH) ; amine primaire (-NH₂) ; amine secondaire (-NHR₁), amine tertiaire (-NR₁R₂) ou amine quaternaire (-N⁺R₁R₂R₃) avec R₁, R₂ et R₃ étant, indépendamment l'un de l'autre, un radical hydrocarboné, linéaire ou ramifié, saturé ou insaturé ayant de 1 à 22 atomes de carbone, sous réserve que la somme des atomes de carbone de R' + R₁ + R₂ + R₃ ne dépasse pas 22, R' pouvant également être un radical perfluoroalkyle, de préférence ayant de 1 à 18 atomes de carbone;
- les groupements -NH₂, -NHR' et -NR'R" dans lesquels R' et R" sont, indépendamment l'un de l'autre des radicaux hydrocarbonés, linéaires, cycliques ou ramifiés, saturés ou insaturés, ayant de 1 à 22 atomes de carbone, sous réserve que le nombre total d'atomes de carbone de R' + R" ne dépasse pas 22, R' et R" étant éventuellement substitués par un atome d'halogène (iode, brome, chlore ou fluor) ; un groupement hydroxyle (-OH) ;sulfonique (-SO₃⁻) ; sulfate (-SO₄⁻) ; phosphate (-PO₄H₂⁻) ; amine primaire (-NH₂); amine secondaire (-NHR₁), amine tertiaire (-NR₁R₂) et/ou amine quaternaire (-N⁺R₁R₂R₃) avec R₁, R₂ et R₃ étant, indépendamment l'un de l'autre, un radical hydrocarboné, linéaire ou ramifié, saturé ou insaturé ayant de 1 à 22 atomes de carbone, sous réserve que la somme des atomes de carbone de R' + R" + R₁ + R₂ + R₃ ne dépasse pas 22, R' et R" pouvant également être des radicaux perfluoroalkyles, de préférence ayant de 1 à 18 atomes de carbone.

19. Composition selon la revendication 18, **caractérisée en ce que** le rapport de concentration en poids entre le composé lipophile et le copolymère bloc amphiphile est compris entre 0,005 et 0,5, de préférence entre 0,005 et 0,2.

20. Composition selon l'une quelconque des revendications 18 à 19, **caractérisée en ce que** le composé lipophile est présent dans la composition en une quantité en poids comprise entre 0,001 et 10% en poids, par rapport au poids total de la composition.

21. Composition selon l'une quelconque des revendications 18 à 20, **caractérisée en ce qu'**elle contient en outre un ou plusieurs adjuvants de formulation choisis parmi les pigments, les nanopigments, les corps gras, les solvants organiques, les épaississants, les adoucissants, les agents anti-radicaux, libres, les stabilisants, les émollients, les silicones, les α-hydroxyacides, les agents anti-mousse, les agents hydratants, les vitamines, les conservateurs, les tensioactifs, les charges, les séquestrants, les polymères, les propulseurs, les agents alcalinisants ou acidifiants, les colorants, les opacifiants.

22. Composition selon l'une quelconque des revendications 18 à 21, **caractérisée en ce qu'**elle est une composition protectrice de l'épiderme humain ou des cheveux contre les rayons ultraviolets, comme les compositions antisolaires; ou est un produit de maquillage; ou est une composition pour protéger les cheveux.
